# EUROPEAN PATENT APPLICATION

(11) **EP 3 825 318 A1**
(43) Date of publication of application: **26.05.2021**
(21) Application number: 19211249.8
(22) Date of filing: 25.11.2019
(51) Int. Cl.: C07D 515/14, A61P 31/20, A61K 31/407

(54) **OXALAMIDO-SUBSTITUTED TRICYCLIC INHIBITORS OF HEPATITIS B VIRUS**

(71) Applicant: Promidis S.r.l., 20132 Milano MI (IT); Ospedale San Raffaele S.r.l., 20132 Milano (MI) (IT); Istituto Nazionale di Genetica Molecolare - INGM, 20122 Milano (MI) (IT); IRBM S.P.A., 00071 Pomezia (RM) (IT)
(72) Inventor: De Francesco, Raffaele, 20122 Milano (MI) (IT); Donnici, Lorena, 20122 Milano (MI) (IT); Di Fabio, Romano, 00071 Pomezia (RM) (IT); Summa, Vincenzo, 00071 Pomezia (RM) (IT); Guidotti, Luca, 20132 Milano (MI) (IT); Iannacone, Matteo, 20132 Milano (MI) (IT); Bencheva, Leda Ivanova, 20132 Milano (MI) (IT); De Matteo, Marilenia, 20132 Milano (MI) (IT); Ferrante, Luca, 20132 Milano (MI) (IT); Prandi, Adolfo, 20132 Milano (MI) (IT); Randazzo, Pietro, 20132 Milano (MI) (IT); Gornati, Davide, 20132 Milano (MI) (IT); Grillo, Alessandro, 20132 Milano (MI) (IT)
(74) Representative: Capasso, Olga

(57) **Abstract**

The present invention relates to compounds that are inhibitors of hepatitis B virus (HBV). Compounds of this invention are useful alone or in combination with other agents for treating, ameliorating, preventing or curing HBV infection and related conditions. The present invention also relates to pharmaceutical compositions containing said compounds.

SEQUENCE LISTING

<110>  PROMIDIS S.R.L.
       IRBM S.P.A.
       OSPEDALE SAN RAFFAELE S.R.L.
       ISTITUTO NAZIONALE DI GENETICA MOLECOLARE - INGM

<120>  OXALAMIDO-SUBSTITUTED TRICYCLIC INHIBITORS OF HEPATITIS B VIRUS

<130>  BE141758

<160>  2

<170>  PatentIn version 3.5

<210>  1
<211>  22
<212>  DNA
<213>  Artificial Sequence

<220>
<223>  synthetic primer

<400>  1
atttgttcag tggttcgtag gg                                              22


<210>  2
<211>  22
<212>  DNA
<213>  Artificial Sequence

<220>
<223>  synthetic primer

<400>  2
cggtaaaaag ggactcaaga tg                                              22

## Description

### FIELD OF THE INVENTION

The present invention relates to compounds that are inhibitors of hepatitis B virus (HBV). Compounds of this invention are useful alone or in combination with other agents for treating, ameliorating, preventing or curing HBV infection and related conditions. The present invention also relates to pharmaceutical compositions containing said compounds.

### BACKGROUND OF THE INVENTION

The Hepatitis B virus (HBV) is an enveloped, partially double-stranded DNA (dsDNA) virus of the hepadnaviridae family that is spread by contact with infected blood and body fluids and causes acute and chronic necroinflammatory liver diseases of varying severity (Guidotti LG, Chisari FV. Annu Rev Pathol. 2006; 1:23-61). The HBV lipid envelope contains 3 in-frame viral envelope proteins (large, middle and small), each of which possesses the hepatitis B virus surface antigen (HBsAg) determinant (Seeger C, Mason WS.Virology. 2015 May; 479-480:672-86). This envelope encloses a protein shell, or capsid, that is composed of 240 monomers of the core protein and each monomer possesses the hepatitis B virus core antigen (HBcAg or Cp) determinant. The capsid in turn encloses a partially double-stranded, relaxed circular DNA (rcDNA) form of the viral genome as well as a molecule of the viral polymerase. Upon entry into susceptible cells (i.e. the hepatocytes) via the interaction of the large envelope protein with specific receptors on the hepatocellular membrane, the capsid is released into the cytoplasm and transported at the nuclear membrane. The rcDNA is then released into the nucleus and repaired by cellular polymerases into an episomal "minichromosome", termed covalently closed circular DNA (cccDNA), which represents the viral transcriptional template. The minus strand of the viral DNA encodes 3.5, 2.4, 2.1 and 0.7 kb mRNA species that are translated into structural (envelope and core) and nonstructural (polymerase, precore and X) proteins of the virus. Following transport into the cytoplasm, one of the 3.5 kb RNAs (termed pregenomic RNA) is selectively packaged into a nascent capsid by interacting with the core and polymerase proteins that have been translated from their respective mRNAs. Within these capsids, the viral polymerase reverse transcribes the pregenomic RNA into a single (-) strand DNA molecule that serves as template for the viral polymerase-mediated DNA (+) strand synthesis and the cohesive structure of the linear DNA intermediates converts them into a relaxed circular double stranded molecule. A fraction of these HBV DNA-containing "mature" capsids are transported back to the nucleus where second strand synthesis is completed and the ends of both strands are ligated, leading to amplification of the pool of cccDNA. Another fraction of the capsids binds to viral envelope proteins that have been independently translated and translocated to membranes of endoplasmic reticulum (ER)-like structures. Following binding, the enveloped capsids bud into the lumen of the ER and exit the cell as infectious virions to initiate new cycles of infection.

Thus, the HBV core protein and the related capsids are essential components and regulators of the HBV life cycle. The full-length core protein Cp183, or its N-terminal domain Cp149, predominantly assembles into a T = 4 icosahedral capsids. Due to its critical roles in capsid assembly, pregenomic RNA packaging, and cccDNA maintenance, it is not surprising that the HBV core protein and the related capsids have been widely recognized as attractive antiviral targets (Durantel D, Zoulim F; J Hepatol. 2016 Apr;64(1 Suppl):S117-S131).

According to World Health Organization (WHO) statistics, HBV infection is one of the major medical scourges of our time. As a sexually transmitted disease that is also transferred by intravenous drug abuse and from mother to infant at birth, over one third of the world's population has been infected by HBV at some point in their lives (Burns GS, Thompson AJ; Cold Spring Harb Perspect Med. 2014 Oct 30;4(12)). While most of these people have successfully cleared the virus, more than 250 million people remain persistently infected and almost 900,000 of these individuals die annually from the complications of chronic infection (i.e. cirrhosis and/or hepatocellular carcinoma). HBV infection is highly endemic in sub-Saharan Africa, the Pacific, and particularly Asia. Regions with high rates of chronic HBV infection also include the Middle East, the Indian subcontinent, areas of South and Central America, and the southern parts of Eastern and Central Europe. In recent years the number of chronic carriers has increased steadily in the western world as well, mostly because of the influx of immigrants from endemic areas. Additionally, HBV acts as a helper virus to hepatitis delta virus (HDV) and it should be noted that the more than 15 million people co-infected with HBV and HDV have an increased risk of rapid progression to cirrhosis and hepatic decompensation (Hughes, S.A. et al. Lancet 2011, 378, 73-85).

Well-tolerated vaccines that elicit neutralizing antibodies to HBsAg efficiently prevent de novo HBV infection, but have no therapeutic potential for the millions of people that are already persistently infected (Zoulim, Durantel D; Cold Spring Harb Perspect Med. 2015 Apr 1;5(4)). Therapy for these individuals mainly relies on direct acting antiviral (DAA) drugs (e.g. tenofovir, lamivudine, adefovir, entecavir or telbivudine) that suppress virus production but do not eradicate HBV from the liver, requiring lifelong treatment. Cohorts of patients still receive a therapy based on pegylated interferon-a (PEG-IFN-α), which has the advantages of limited treatment duration and higher rates of HBsAg seroconversion but the relevant disadvantage of greater adverse effects. As such, the number of patients receiving PEG-IFN-α is progressively decreasing.

Different chemical classes of inhibitors targeting the encapsidation process of HBV (also termed capsid assembly modulators or CAMs) are under development, and they include heteroaryldihydropyrimidines (HAPs) and sulfamoylbenzamides (SBAs). For instance, Novira Therapeutics recently utilized a humanized mouse model of HBV infection to show that a combination of CAM and PEG-IFN-α has higher antiviral activity than that previously observed with DAAs. NVR3-778, the first member of this class of CAM, in Phase 1b proof-of-concept clinical studies showed both significant reduction in HBV DNA and serum HBV RNA. This compound was recently discontinued. The compound JNJ-56136379 (or JNJ-379), developed by Janssen, has recently demonstrated potent antiviral activity and is now entering into Phase 2 clinical trial.

WO2013/006394, published on January 10, 2013, relates to a subclass of sulfamoyl-arylamides having general formula A, useful for the treatment of Hepatitis B virus (HBV) infection:

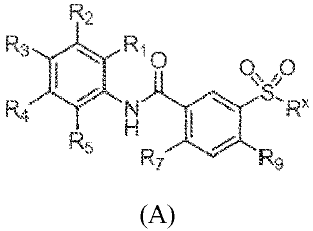

WO2013/096744, published on June 26, 2013 relates to sulfamoyl-arylamides of formula B active against HBV:

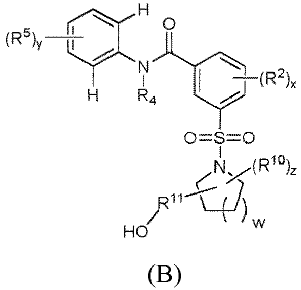

WO2014/106019, published on July 3, 2014, relates to compounds of formula C, useful as nucleocapsid assembly inhibitors for the treatment of viruses, especially but not exclusively, including pregenomic RNA encapsidation inhibitors of HBV for the treatment of Hepatitis B virus (HBV) infection and related conditions:

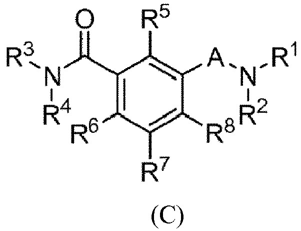

WO2014/165128, published on October 9, 2014, WO2015/109130 published on July 23, 2015, US2015274652, published on October 1, 2015, all relate to sulfamoyl-arylamides compounds active against HBV.

WO2015/120178, published on August 13, 2015, relates to sulfamoyl-arylamides compounds used in combinantion therapy with peginterferon alfa-2a, or another interferon analog for the treatment of HBV infection.

WO2016/089990, published on June 9, 2016, relates to sulfide alkyl and pyridyl reverse sulphonamide compounds for HBV treatment.

US2016185748, published on June 30, 2016, relates to pyridyl reverse sulfonamides for HBV treatment.

US2016151375, published on June 2, 2016 relates to sulfide alkyl compounds for HBV treatment. WO2017/001655A1, published on January 5, 2017, relates to cyclized sulfamoylarylamide derivatives having structure:

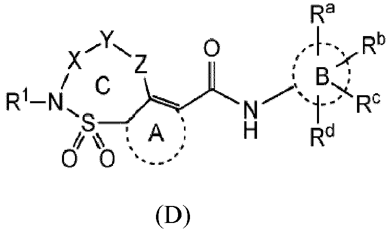

Amongst the problems which HBV direct antivirals may encounter are toxicity, mutagenicity, lack of selectivity, poor efficacy, poor bioavailability, low solubility and/or off-target activity, and until now there are no compounds in any of the structural classes identified above approved as drugs for the treatment of HBV patients.

There is a need for additional HBV inhibitors that may overcome at least one of these disadvantages or that have additional advantages such as increased potency, increased bioavailability or an increased safety window.

The present invention provides small molecule drugs obtained through chemical modification of the known sulfamoyl arylamides derivatives. In particular the compounds of the invention are characterized by a fused tricyclic core structure comprising a pyrrole ring, bearing an oxalamide substituent on a specific position of the fused tricyclic core. The chemotype discovered in the present invention results in extremely potent HBV inhibitors with improved pharmacokinetic properties, good kinetic solubility, stability in mouse and human hepatocytes, low in vivo clearance and positive liver-to-plasma concentration. Given the liver's key role in metabolic regulation and the fact that it is the principal tissue affected by hepatitis B disease, designing HBV inhibitors with hepatoselective distribution profiles is an important strategy in developing safe drug candidates (Tu M. et al., Current Topics in Medicinal Chemistry, 2013, 13, 857-866).

### DESCRIPTION OF THE INVENTION

The compounds of this invention are inhibitors of hepatitis B virus (HBV).

It is therefore an object of the present invention a compound of Formula (I): 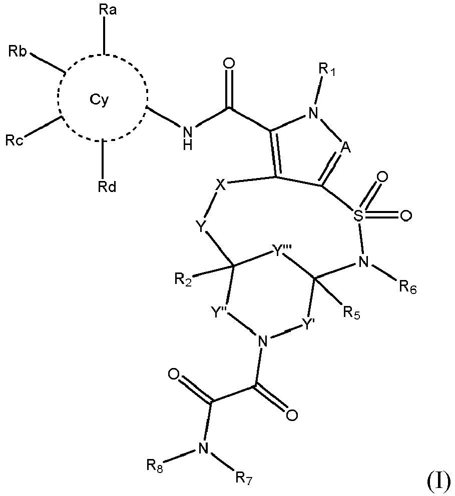 wherein:
Cy is aryl or heteroaryl;
A is C-R₃ or N;
X is O, S, NH, SO, SO₂ or a single bond;
Y, Y', Y" and Y'" are each independently C₁-₆alkanediyl or C₂₋₇alkenediyl, each optionally substituted with one or more R₄, or a single bond;
R₁ is H or C₁₋₆alkyl;
R₂ is selected from H, OH and C₁₋₆alkyl;
R₃ is selected from H, C₁₋₆alkyl, C₃₋₈cycloalkyl, haloC₁₋₆alkyl and halogen;
R₄ is selected from H, OH, C₁₋₆alkyl, C₃₋₈cycloalkyl and halogen or two geminal R₄ form together with the atom to which they are attached a spiro-C₃₋₈cycloalkyl or a spiro-C₃₋₈heterocycloalkyl; R₅ is H or C₁₋₆alkyl;
or R₂ and R₅ taken together form a C₁₋₆alkanediyl bridge;
R₆ is selected from H, C₁₋₆alkyl, C₃₋₈cycloalkyl, C₃₋₈heterocycloalkyl, C₁₋₆alkylaryl, C₁₋₆alkylheteroaryl and C₁₋₆alkyl-C₃₋₈cycloalkyl wherein each of said C₁₋₆alkyl, C₃₋₈cycloalkyl, C₃₋₈heterocycloalkyl, C₁₋₆alkylaryl, C₁₋₆alkylheteroaryl or C₁₋₆alkyl-C₃₋₈cycloalkyl is optionally substituted with one or more substituents each independently selected from: OH, halogen, haloC₁₋₆alkyl, cyano and NH₂;
each of R₇ and R₈ are independently selected from:
   - hydrogen;
   - C₁₋₁₂alkyl optionally substituted with one or more substituents each independently selected from the group consisting of: OH, halogen, CN, NH₂, NH(R₉), N(R₉)₂, haloC₁-₆alkyl, aryl, heteroaryl, 3-7 membered saturated ring and 5-7 membered partially saturated ring, each of said saturated or partially saturated ring optionally containing one or more heteroatoms selected from the group consisting of O, N and S and each of said aryl, heteroaryl, 3-7 membered saturated ring or 5-7 partially saturated ring being optionally substituted with one or more substituents each independently selected from: OH, halogen, C₁₋₆alkyl, haloC₁₋₆alkyl, CN, haloC₁₋₆alkoxy and C₁₋₆alkoxy;
   - aryl or heteroaryl, each of said aryl or heteroaryl being optionally substituted with one or more substituents each independently selected from: OH, halogen, haloC₁₋₆alkyl, CN, haloC₁₋₆alkoxy and C₁₋₆alkoxy; and
   - a 3-8 membered saturated or partially saturated cyclic or bicyclic ring optionally containing one or more heteroatoms each independently selected from the group consisting of: O, S and N, the 3-8 membered saturated or partially saturated cyclic or bicyclic ring being optionally substituted with one, two or more substituents each independently selected from the group consisting of: OH, halogen, CN, C₁-₆alkyl, hydroxyC₁-₆alkyl, C(O)OR₉, C(O)R₉, haloC₁₋₆alkyl, haloC₁₋₆alkoxy and C₁₋₆alkoxy;
or R₇ and R₈ form together with the nitrogen atom to which they are attached a cyclic amine selected from: aziridine, azetidine, pyrrolidine, piperidine, azepane, morpholine, thiomorpholine and piperazine each of said cyclic amine being optionally substituted with one or more substituents each independently selected from the group consisting of: OH, halogen, CN, C₁-₆alkyl, hydroxyC₁-₆alkyl, haloC₁₋₆alkyl, haloC₁₋₆alkoxy and C₁₋₆alkoxy;
Ra, Rb, Rc and Rd are each independently selected from the group consisting of: hydrogen, halogen, CN, C₁-₆alkyl, C₁-₆alkoxy, haloC₁-₆alkyl, haloC₁-₆alkoxy, C(O)OR₉, C(O)R₉, NH₂, NH(R₉) and N(R₉)₂;
each R₉ is independently selected from C₁-₆alkyl, haloC₁-₆alkyl, C₁₋₆alkylaryl, C₁₋₆alkylheteroaryl and C₁₋₆alkyl-C₃₋₈cycloalkyl;
or a pharmaceutically acceptable salt, tautomer, solvate or stereoisomer thereof.

Preferably, R₂ is H or C₁₋₆alkyl and R₃ is H, C₁₋₆alkyl or halogen.

In one embodiment, the invention relates to compounds of Formula (I-A): 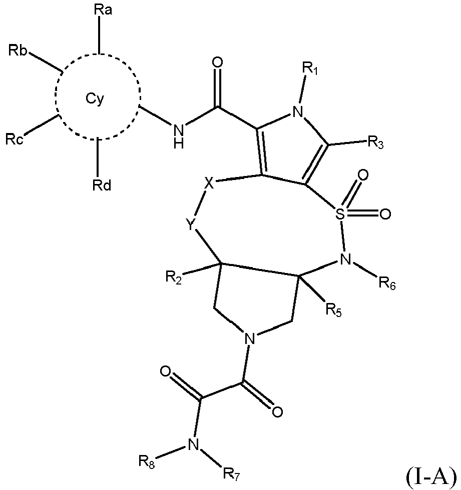 or Formula (I-B): 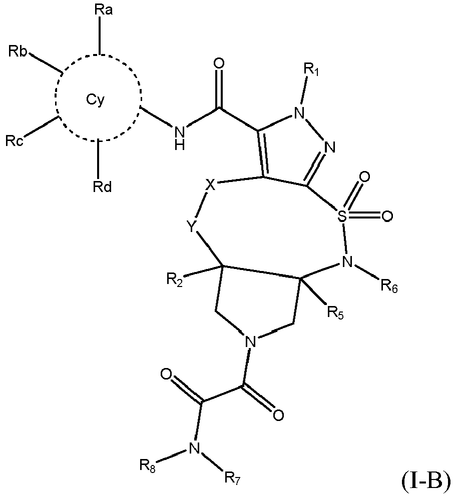 or pharmaceutically acceptable salts, tautomers, solvates or stereoisomers thereof. In particular, in Formula I-A, R₂ is H or C₁₋₆alkyl; R₃ is H, C₁₋₆alkyl or halogen and the remaining substituents are as defined above.

In a preferred embodiment, compounds of the invention have Formula II-A: 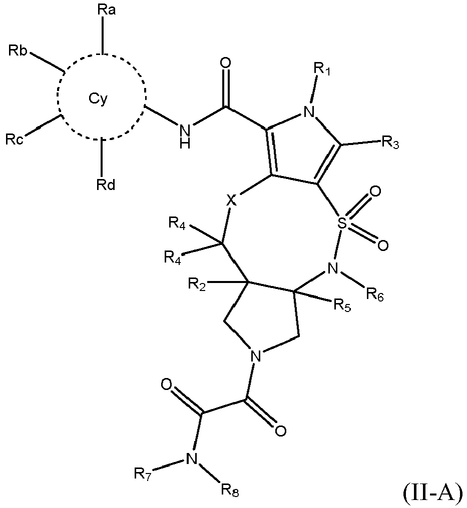 wherein:
X is O, S, NH, SO, SO₂;
R₂ is H or C₁₋₆alkyl;
R₃ is H, C₁₋₆alkyl or halogen;
each R₄ is independently selected from H, OH, C₁₋₆alkyl, C₃₋₈cycloalkyl and halogen or two R₄ form together with the atom to which they are attached a spiro-C₃₋₈cycloalkyl or a spiro-C₃₋₈heterocycloalkyl;
the remaining substituents being as defined above;
or pharmaceutically acceptable salts, tautomers, solvates or stereoisomers thereof.

An additional embodiment of the present invention relates to compounds of Formula (II-B): 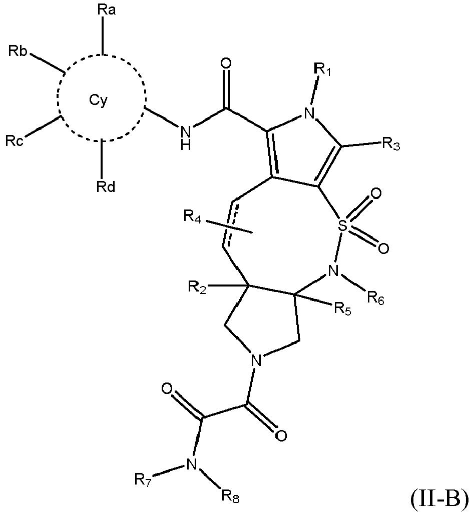 wherein:
-̅-̅-̅-̅-̅-̅ represents a single or double bond;
R₂ is H or C₁₋₆alkyl;
R₃ is H, C₁₋₆alkyl or halogen;
or pharmaceutically acceptable salts, tautomers, solvates or stereoisomers thereof.

In a further preferred embodiment in the compounds of Formula (I), Formula (I-A), Formula (I-B), Formula (II-A) or Formula (II-B), or in the pharmaceutically acceptable salts, tautomers, solvates or stereoisomers thereof, Cy is phenyl, R₁ is CH₃, R₅ is hydrogen and R₆ is hydrogen. Another embodiment of the present invention relates to those compounds of Formula (I), Formula (I-A), Formula (I-B), Formula (II-A) or Formula (II-B), or to the pharmaceutically acceptable salts, tautomers, solvatse or stereoisomers thereof, wherein:
R₇ is selected from the group consisting of hydrogen, methyl, ethyl, isopropyl, *tert*-butyl, cyclopropyl, cyclobutyl 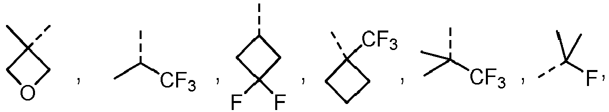 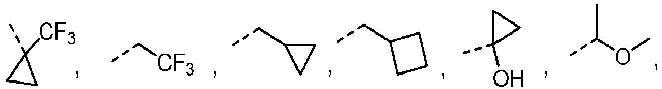 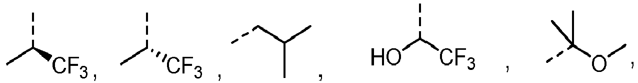 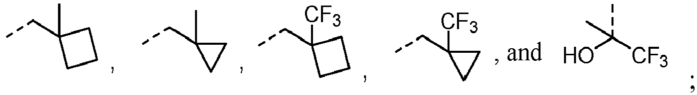 and/or
R₈ is selected from the group consisting of hydrogen, methyl, ethyl, isopropyl, *tert*-butyl, cyclopropyl, cyclobutyl

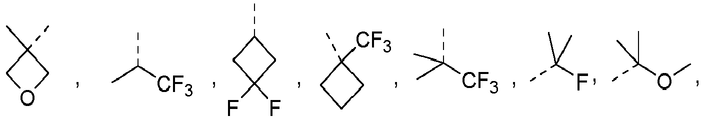

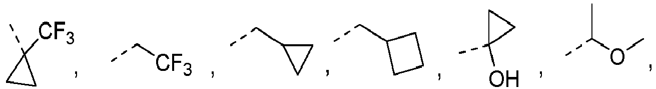

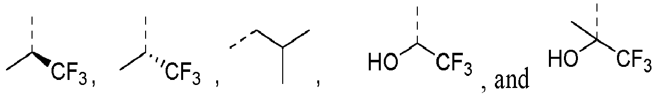

Preferably, in any of the compounds, pharmaceutically acceptable salts, tautomers, solvates or stereoisomers thereof as defined above, Cy is aryl (in particular phenyl).

Preferably, in any of the compounds of formula (I), or in the pharmaceutically acceptable salts, tautomers, solvates or stereoisomers thereof as defined above, A is C-R₃.

Preferably, in any of the compounds of formula (I), (I-A), (I-B) or (II-A), or in the pharmaceutically acceptable salts, tautomers, solvates or stereoisomers thereof as defined above X is O or S.

Preferably, in any of the compounds of formula (I), (I-A) or (I-B), or in the pharmaceutically acceptable salts, tautomers, solvates or stereoisomers thereof as defined above Y is C₁₋₆alkanediyl (in particular methanediyl).

Preferably, in any of the compounds of formula (I) or in the pharmaceutically acceptable salts, tautomers, solvates or stereoisomers thereof as defined above Y' is C₁₋₆alkanediyl (in particular methanediyl).

Preferably, in any of the compounds of formula (I) or in the pharmaceutically acceptable salts, tautomers, solvates or stereoisomers thereof as defined above Y" is C₁₋₆alkanediyl (in particular methanediyl).

Preferably, in any of the compounds of formula (I) or in the pharmaceutically acceptable salts, tautomers, solvates or stereoisomers thereof as defined above Y''' is a single bond.

Preferably, in any of the compounds of formula (I), or in the pharmaceutically acceptable salts, tautomers, solvates or stereoisomers thereof as defined above Y, Y', Y" are C₁₋₆alkanediyl (in particular methanediyl) and Y''' is a single bond.

Preferably, in any of the compounds, pharmaceutically acceptable salts, tautomers, solvates or stereoisomers thereof as defined above R₁ is C₁-₆alkyl (in particular methyl).

Preferably, in any of the compounds, pharmaceutically acceptable salts, tautomers, solvates or stereoisomers thereof as defined above R₂ is H or C₁-₆alkyl (in particular methyl).

Preferably, in any of the compounds of formula (I), (I-A), (II-A) or (II-B) or in the pharmaceutically acceptable salts, tautomers, solvates or stereoisomers thereof as defined above R₃ is H, methyl or chlorine.

Preferably, in any of the compounds, pharmaceutically acceptable salts, tautomers, solvates or stereoisomers thereof as defined above R₅ is H or C₁-₆alkyl (in particular methyl);

Preferably, in any of the compounds, pharmaceutically acceptable salts, tautomers, solvates or stereoisomers thereof as defined above R₆ is H.

Preferably, in any of the compounds, pharmaceutically acceptable salts, tautomers, solvates or stereoisomers thereof as defined above R₇ and R₈ are independently selected from:
- hydrogen,
- C₁₋₁₂alkyl (in particular C₁₋₄alkyl, preferably methyl, ethyl, *i*-propyl, *t*-butyl or *i*-butyl) optionally substituted with one or more substituents each independently selected from: halogen (in particular fluorine), haloC₁-₆alkyl (in particular trifluoromethyl) and 3-7 membered saturated ring optionally containing an oxygen atom (in particular oxetanyl, cyclopropyl or cyclobutyl), and
- 3-8 membered saturated or partially unsaturated cyclic or bicyclic ring optionally containing an oxygen atom (in particular oxetanyl, cyclopropyl or cyclobutyl) optionally substituted with one or more substituents each independently selected from: C₁-₆alkyl (in particular methyl), halogen (in particular fluorine) and haloC₁-₆alkyl (in particular trifluoromethyl),
or R₇ and R₈ form together with the nitrogen atom to which they are attached an azetidine optionally substituted with one or more halogens (in particular fluorine).

Preferably, in any of the compounds, pharmaceutically acceptable salts, tautomers, solvates or stereoisomers thereof as defined above, Ra, Rb, Rc, and Rd are each independently selected from the group consisting of: hydrogen, halogen (in particular fluorine or chlorine), C₁-₆alkyl (in particular methyl) and CN.

In a preferred embodiment, the present invention provides a compound of general formula (I) as defined above, wherein:
Cy is aryl (in particular phenyl);
A is C-R₃;
X is O or S;
Y, Y', Y" are C₁-₆alkanediyl (in particular methanediyl);
Y''' is a single bond;
R₁ is C₁-₆alkyl (in particular methyl);
R₂ is H or C₁-₆alkyl (in particular methyl);
R₃ is H, methyl or chlorine;
R₅ is H or C₁-₆alkyl (in particular methyl);
R₆ is H;
R₇ and R₈ are independently selected from:
   - hydrogen,
   - C₁₋₁₂alkyl (in particular C₁₋₄alkyl, preferably methyl, ethyl, *i*-propyl, *t*-butyl or *i*-butyl) optionally substituted with one or more substituents each independently selected from: halogen (in particular fluorine), haloC₁-₆alkyl (in particular trifluoromethyl) and 3-7 membered saturated ring optionally containing an oxygen atom (in particular oxetanyl, cyclopropyl or cyclobutyl), and
   - 3-8 membered saturated or partially unsaturated cyclic or bicyclic ring optionally containing an oxygen atom (in particular oxetanyl, cyclopropyl or cyclobutyl) optionally substituted with one or more substituents each independently selected from: C₁-₆alkyl (in particular methyl), halogen (in particular fluorine) and haloC₁-₆alkyl (in particular trifluoromethyl),
or R₇ and R₈ form together with the nitrogen atom to which they are attached an azetidine optionally substituted with one or more halogens (in particular fluorine);
Ra, Rb, Rc, and Rd are each independently selected from the group consisting of: hydrogen, halogen (in particular fluorine or chlorine), C₁-₆alkyl (in particular methyl) and CN.

The compounds of the invention are selected from the group consisting of:
- (3aR,10aR)-N-(3-chloro-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-N-(3,4-difluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-N-(3-(difluoromethyl)-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-N-(4-fluoro-3-(trifluoromethyl)phenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-6-chloro-N-(3-chloro-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-6-chloro-N-(3,4-difluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-6-chloro-N-(3-(difluoromethyl)-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-6-chloro-N-(4-fluoro-3-(trifluoromethyl)phenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-6-chloro-N-(4-fluoro-3-methylphenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
or a pharmaceutically acceptable salt, tautomer, solvate or stereoisomer thereof.

Preferably, the compounds or the pharmaceutically acceptable salts, tautomers, solvates, or stereoisomers thereof as defined above are for medical use. Still preferably, the compounds or the pharmaceutically acceptable salts, tautomers, solvates, or stereoisomers thereof as defined above are for use in the treatment and/or prevention of an HBV infection and/or a condition related to an HBV infection. Preferably, said condition related to an HBV infection is selected from the group consisting of: chronic hepatitis B, HBV/HDV co-infection, HBV/HCV co-infection, HBV/HIV co-infection, inflammation, necrosis, cirrhosis, hepatocellular carcinoma, hepatic decompensation and hepatic injury from an HBV infection.

Even more preferably, the compounds of the invention or the pharmaceutically acceptable salts, tautomers, solvates, or stereoisomers thereof are for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection.

Preferred compounds exhibit an HBV inhibition greater than 50% at the test concentration (ranging from 1.0 micromolar to 0.1 micromolar) and/or an EC₅₀, as defined hereinafter, lower than 0.5 micromolar. HBV inhibition indicates inhibition of HBV expression and replication. The inhibition activity of the compounds of the invention can be measured as described hereinafter. Preferably, the compound or the pharmaceutically acceptable salt, tautomer, solvate, or stereoisomer thereof as defined above is for use in combination with at least one further therapeutic agent. Preferably, said use in combination comprises the administration of at least one further therapeutic agent.

It is an object of the invention a pharmaceutical composition comprising the compound or the pharmaceutically acceptable salt, tautomer, solvate, or stereoisomer thereof as defined above, alone or in combination with at least one further therapeutic agent, and at least one pharmaceutically acceptable excipient.

Preferably, the at least one further therapeutic agent is selected from the group consisting of: a therapeutic vaccine; an RNA interference therapeutic/antisense oligonucleotide; an immunomodulator; a STING agonist; a RIG-I modulator; a NKT modulator; an IL agonist; an interleukin or another immune acting protein; a therapeutic and prophylactic vaccine; an immune checkpoint modulator/inhibitor; an HBV entry inhibitor; a cccDNA modulator; an inhibitor of HBV protein espression; an agent targeting HBV RNA; a capsid assembly inhibitor/modulator; a core or X protein targeting agent; a nucleotide analogue; a nucleoside analogue; an interferon or a modified interferon; an HBV antiviral of distinct or unknown mechanism; a cyclophilin inhibitor; a sAg release inhibitor; a HBV polymerase inhibitor; a dinucleotide; a SMAC inhibitor; a HDV targeting agent; a viral maturation inhibitor; a reverse transcriptase inhibitor and an HBV RNA destabilizer or another small-molecule inhibitor of HBV protein expression; or a combination thereof.

Preferably, the therapeutic vaccine is selected from: HBsAG-HBIG, HB-Vac, ABX203, NASVAC, GS-4774, GX- 110 (HB-110E), CVI-HBV-002, RG7944 (INO-1800), TG-1050, FP-02 (Hepsyn-B), AIC649, VGX-6200, KW-2, TomegaVax-HBV, ISA-204, NU-500, INX-102-00557, HBV MVA and PepTcell.

Preferably, the RNA interference therapeutic is a siRNA, a ddRNA or a shRNA. Preferably, the RNA interference therapeutic is selected from: TKM-HBV (ARB-1467), ARB-1740, ARC-520, ARC-521, BB-HB-331, REP-2139, ALN-HBV, ALN-PDL, LUNAR-HBV, GS3228836 and GS3389404.

Preferably, the immunomodulator is a TLR agonist. Preferably the TLR agonist is a TLR7, TLR8 or TLR9 agonist. Preferably, the TLR7, TLR8 or TLR9 agonist is selected from: RG7795 (RO-6864018), GS-9620, SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-pyrin-9-yl)propyl][3-(4-morpholinyl)propyl]amino}methyl)phenyl]acetate) and ARB-1598.

Preferably, the RIG-I modulator is SB-9200. Preferably, the IL agonist or other immune acting protein is INO-9112 or recombinant IL12. Preferably, the immune checkpoint modulator/inhibitor is BMS-936558 (Opdivo (nivolumab)) or pembrolizumab. Preferably, the HBV entry inhibitor is Myrcludex B, IVIG-Tonrol or GC-1102.

Preferably, the cccDNA modulator is selected from: a direct cccDNA inhibitor, an inhibitor of cccDNA formation or maintenance, a cccDNA epigenetic modifier and an inhibitor of cccDNA transcription.

Preferably, the capsid assembly inhibitor/modulator, core or X protein targeting agent, direct cccDNA inhibitor, inhibitor of cccDNA formation or maintenance, or cccDNA epigenetic modifier is selected from: BAY 41-4109, NVR 3-778, GLS-4, NZ-4 (W28F), Y101, ARB-423, ARB-199, ARB-596, AB-506, JNJ-56136379, ASMB-101 (AB-V102), ASMB-103, CHR-101, CC-31326, AT-130 and RO7049389.

Preferably, the interferon or modified interferon is selected from: interferon alpha (IFN-α), pegylated interferon alpha (PEG-IFN-a), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta-la, peginterferon beta-la, interferon delta, interferon lambda (IFN-λ), peginterferon lambda-1, interferon omega, interferon tau, interferon gamma (IFN-γ), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA and interferon alpha biobetter. Particularly preferred are: peginterferon alpha-2a, peginterferon alpha-2b, glycosylated interferon alpha-2b, peginterferon beta-la, and peginterferon lambda-1. More particularly preferred is peginterferon alpha-2a. Preferably, the HBV antiviral of distinct or unknown mechanism is selected from: AT-61 ((E)-N-(1 -chloro-3-oxo-1 -phenyl-3 -(piperidin-1 -yl)prop-1 -en-2-yl)benzamide), AT 130 ((E)-N-(1-bromo-1 -(2-methoxyphenyl)-3 -oxo-3-(piperidin-1 -yl)prop-1 -en-2-yl)-4-nitrobenzamide), analogues thereof, REP-9AC (REP-2055), REP-9AC' (REP-2139), REP-2165 and HBV-0259. Preferably, the cyclophilin inhibitor is selected from: OCB-030 (NVP-018), SCY-635, SCY-575 and CPI-431-32.

Preferably, said HBV polymerase inhibitor is selected from: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir. Preferably, tenofovir is in a salt form. Preferably, tenofovir is in a salt form selected from: tenofovir disoproxil fumarate (Viread), tenofovir alafenamide fumarate (TAF), tenofovir disoproxil orotate (DA-2802), tenofovir disopropxil aspartate (CKD-390), AGX-1009, and CMX157.

Preferably, the dinucleotide is SB9200. Preferably, the SMAC inhibitor is Birinapant. Preferably, the HDV targeting agent is Lonafamib.

Preferably, the HBV RNA destabilizer or other small-molecule inhibitor of HBV protein expression is RG7834 or AB-452.

Preferably, the at least one further therapeutic agent is an agent useful in the treatment and prevetion of hepatitis B. Preferably, the at least one further therapeutic agent is an anti-HDV agent, an anti-HCV agent and/or an anti-HIV agent.

Preferably, the at least one further therapeutic agent is selected from the group consisting of: HBV polymerase inhibitor, interferon, viral entry inhibitor, BAY 41-4109, reverse transcriptase inhibitor, a TLR-agonist, AT-61 ((E)-N-(1-chloro-3-oxo-1-phenyl-3-(piperidin-1-yl)prop-1-en-2-yl)benzamide), AT-130 ((E)-N-(1-bromo-1-(2-methoxyphenyl)-3-oxo-3-(piperidin-1-yl)prop-1-en-2-yl)-4-nitrobenzamide), and a combination thereof, wherein the HBV polymerase inhibitor is preferably at least one of Lamivudine, Entecavir, Tenofovir, Adefovir, Telbivudine, Clevudine; and wherein the TLR agonist is preferably selected from the group consisting of SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-purin-9-yl)propyl][3-(4-morpholinyl)propyl] amino }methyl)phenyl] acetate) and a combination thereof.

Preferably, the compound of the invention is for use in combination with one, two or more further therapeutic agent(s) as defined above.

Preferably, the pharmaceutical composition of the invention comprises one, two or more further therapeutic agent(s) as defined above.

Preferably, said pharmaceutical composition is for use in the treatment and/or prevention of an HBV infection and/or a condition related to an HBV infection, said condition related to an HBV infection being preferably selected from the group consisting of: chronic hepatitis B, HBV/HDV co-infection, HBV/HCV co-infection, HBV/HIV co-infection, inflammation, necrosis, cirrhosis, hepatocellular carcinoma, hepatic decompensation and hepatic injury from an HBV infection. Even more preferably, said pharmaceutical composition is for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection. In an embodiment, the invention provides a kit comprising at least one pharmaceutically acceptable vial or container containing one or more doses of a compound of the invention or of a pharmaceutical composition of the invention and optionally a) instructions for use thereof in mammals and/or b) an infusion bag or container containing a pharmaceutically acceptable diluent. It is a further object of the invention a process for the synthesis of the compounds of the invention comprising at least one of the following steps:
- reacting a compound of formula (1) wherein Ra is selected from the group consisting of Cl, F, CHF₂, CF₃ and CH₃, with a compound of formula (2) in the presence of an amine as 2,3,4,6,7,8,9,10-octahydropyrimido[1,2-a]azepine and in a solvent as ethanol to afford a compound of formula (3): 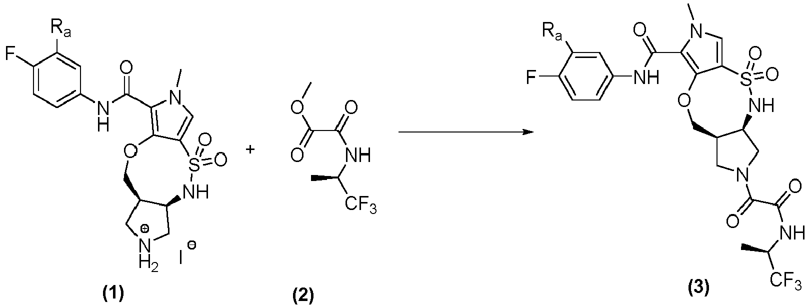
- reacting a compound of formula (3) wherein Ra is selected from the group consisting of Cl, F, CHF₂, CF₃ and CH₃ with sulfuryl dichloride in a solvent like dichloromethane to afford a compound of formula (4):

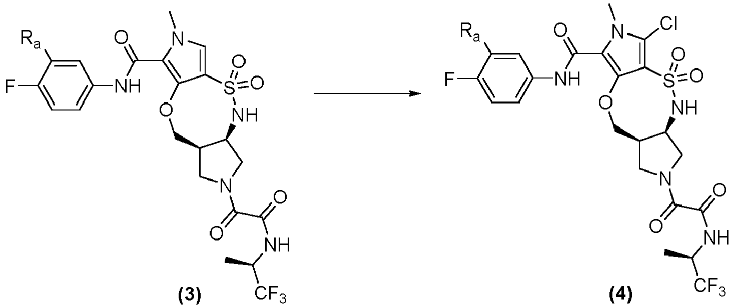

It is a further object of the invention a pharmaceutical composition comprising an effective amount of one or more compounds as defined above or a pharmaceutically acceptable prodrug thereof, alone or in combination with other active compounds, and at least one pharmaceutically acceptable excipient.

The present invention includes within its scope prodrugs of the compounds of the invention which are readily convertible *in vivo* into the required compound of formula (I) as indicated above. Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The invention also includes all suitable isotopic variations of a compound of the disclosure. Examples of isotopes that can be incorporated into compounds of the disclosure include isotopes such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the disclosure, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the compounds of the disclosure can generally be prepared by conventional procedures such as by the illustrative methods or by the preparations described in the examples hereafter using appropriate isotopic variations of suitable reagents. The present invention includes within its scope solvates of the compounds of the invention or of the relative salts, for example, hydrates.

In addition, the compounds disclosed herein may exist as tautomers and all tautomeric forms are intended to be encompassed by the scope of the invention, even though only one tautomeric structure is depicted.

The compounds may exist in different isomeric forms, all of which are encompassed by the present invention.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention.

Pure stereoisomeric forms of the compounds and intermediates of this invention may be obtained by the application of art-known procedures and are intended to be encompassed by the scope of the invention. In particular, "pure stereoisomeric form" or "stereoisomerically pure" indicate a compound having stereoisomeric excess of at least 80%, preferably of at least 85%. For instance, enantiomers may be separated from each other by the selective crystallization of their diastereomeric salts or by chromatographic techniques using chiral stationary phases. Pure stereochemically isomeric forms may also be derived from the corresponding pure stereochemically isomeric forms of the appropriate starting materials, provided that the reaction occurs stereospecifically. The term "enantiomerically pure" shall be interpreted in a similar way, having regard to the enantiomeric ratio.

When any variable (e.g. R₁ and R₂, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is polycyclic, it is intended that the bond be attached to any of the suitable carbon atoms on the proximal ring only.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents.

The expressions "one or more substituents" and "one, two or more substituents" refer to in particular to 1, 2, 3, 4 or more substituents, in particular to 1, 2, 3 or 4 substituents, more in particular 1, 2 or 3 substituents.

As used herein "Y is a single bond" indicates that, in the general Formula (I) X is directly linked via a single bond to the carbon atom bearing R₂; "Y' is a single bond" indicates that, in the general Formula (I), Z is directly linked via a single bond to N; "Y" is a single bond" indicates that, in the general Formula (I), N is directly linked via a single bond to the carbon atom bearing R₂; "Y''' is a single bond" indicates that Z, in the general Formula (I), is directly linked via a single bond to the carbon atom bearing R₂.

As used herein, "alkyl" is intended to include both branched and straight-chain saturated aliphatic hydrocarbon groups having the specified number of carbon atoms. For example, "C₁-₁₂alkyl" is defined to include groups having 1, 2, 3, 4, 5, 6, 7, 8, 9, 10, 11 or 12 carbons in a linear or branched arrangement and specifically includes methyl, ethyl, *n*-propyl, *i*-propyl, *n*-butyl, *t*-butyl, *i*-butyl, pentyl, hexyl, and so on. As another example, "C₁-₆alkyl" is defined to include groups having 1, 2, 3, 4, 5 or 6 carbons in a linear or branched arrangement and specifically includes methyl, ethyl, n-propyl, *i*-propyl, *n*-butyl, *t*-butyl, *i*-butyl, pentyl, hexyl, and so on. Preferably, "C₁-₁₂alkyl" and "C₁-₆alkyl" refer to "C₁-₄alkyl" or "C₁-₃alkyl". "C₁-₄alkyl" is defined to include groups having 1, 2, 3 or 4 carbons in a linear or branched arrangement. For example, "C₁-₄ alkyl" specifically includes methyl, ethyl, n-propyl, *i*-propyl, *n*-butyl, *t*-butyl, *i*-butyl, and so on. "C₁₋₃alkyl" is defined to include groups having 1, 2, or 3 carbons in a linear or branched arrangement. For example, "C₁-₃ alkyl" specifically includes methyl, ethyl, *n*-propyl, *i*-propyl, and so on. Preferred alkyl groups are methyl, ethyl, *i*-propyl, *t*-butyl or *i*-butyl.

As used herein, "alkoxy" represents an alkyl group of indicated number of carbon atoms attached through an oxygen bridge. "Alkoxy" therefore encompasses the definitions of alkyl above. C₁-₆ alkoxy group is preferably a linear or branched C₁-₄ alkoxy group, more preferably a C₁-₃alkoxy group, still more preferably a C₁-₂ alkoxy group. Examples of suitable alkoxy groups include, but are not limited to methoxy, ethoxy, *n*-propoxy, *i*-propoxy, *n*-butoxy, *s*-butoxy or *t*-butoxy. preferred alkoxy groups include methoxy, ethoxy and *t*-butoxy.

As used herein, the terms "haloC₁₋₆alkyl" and "haloC₁₋₆alkoxy" mean a C₁₋₆alkyl or C₁₋₆alkoxy group in which one or more (in particular, 1 to 3) hydrogen atoms have been replaced by halogen atoms, especially fluorine or chlorine atoms. HaloC₁-₆alkoxy group is preferably a linear or branched haloC₁-₄alkoxy group, more preferably a haloC₁-₃alkoxy group, still more preferably a haloC₁-₂alkoxy group, for example OCF₃, OCHF₂, OCH₂F, OCH₂CH₂F, OCH₂CHF₂ or OCH₂CF₃, and most especially OCF₃ or OCHF₂. HaloC₁-₆alkyl group is preferably a linear or branched haloC₁-₃alkyl group, more preferably a haloC₁-₂alkyl group for example, CF₃, CHF₂, CH₂F, CH₂CH₂F, CH₂CHF₂, CH₂CF₃ or CH(CH₃)CF₃, and most especially CF₃, CHF₂ or CH(CH₃)CF₃.

As used herein, the term "hydroxyC₁₋₆alkyl" means a C₁₋₆alkyl group in which one or more (in particular, 1 to 3) hydrogen atoms have been replaced by hydroxy groups. Similarly, the term "hydroxyC₁₋₄alkyl" means a C₁₋₄alkyl group in which one or more (in particular, 1 to 2) hydrogen atoms have been replaced by hydroxy groups. Illustrative examples include, but are not limited to CH₂OH, CH₂CH₂OH, CH(CH₃)OH and CHOHCH₂OH.

As used herein, the term "aryl" means a monocyclic or polycyclic aromatic ring comprising carbon atoms and hydrogen atoms. If indicated, such aromatic ring may include one or more heteroatoms, then also referred to as "heteroaryl", preferably, 1 to 3 heteroatoms, independently selected from nitrogen, oxygen, and sulfur, preferably nitrogen. As is well known to those skilled in the art, heteroaryl rings have less aromatic character than their all-carbon counter parts. Thus, for the purposes of the present invention, a heteroaryl group need only have some degree of aromatic character. Illustrative examples of aryl groups are optionally substituted phenyl. Illustrative examples of heteroaryl groups according to the invention include optionally substituted thiophene, oxazole, thiazole, thiadiazole, imidazole, pyrazole, pyrimidine, pyrazine and pyridine. Thus, examples of monocyclic aryl optionally containing one or more heteroatoms, for example one or two heteroatoms, are a 5- or 6-membered aryl or heteroaryl group such as, but not limited to, phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl. Examples of polycyclic aromatic ring, optionally containing one or more heteroatoms, for example one or two heteroatoms, are a 8-10 membered aryl or heteroaryl group such as, but not limited to, benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzodioxolyl, benzoxadiazolyl, benzoisoxazolyl, benzoisothiazolyl, indolyl, indolizinyl, isoindolinyl, indazolyl, isobenzofuranyl, isoindolyl, isoquinolyl, quinazolinyl, quinolyl, quinoxalinyl, quinolizinyl, naphtyl, naphthyridinyl and phthalazinyl. A preferred aryl according to the present invention is phenyl. A preferred heteroaryl according to the present invention is pyridyl.

Heterocycle, heterocyclic compound or ring structure or heterocycloalkyl is a cyclic compound that has atoms of at least two different elements as members of its ring(s).

As used herein, the term "C₃₋₈heterocycloalkyl" is a saturated or partially saturated non aromatic monocyclic or bicyclic ring system, of 3 to 8 members which contains one or more heteroatoms selected from N, O or S. Examples include, but are not limited to azetidinyl, piperazinyl, piperidinyl, morpholinyl, thiomorpholinyl, thiazolidinyl, pyrrolidinyl, azepanyl, diazepanyl, oxazepanyl, thiazepanyl, azocanyl, oxazocanyl and the hexahydrofuro[2,3-b]furan system.

A substituent on a saturated, partially saturated or unsaturated heterocycle can be attached at any substitutable position.

As used herein, the term "C₁₋₆ alkanediyl" as group or part of a group defines bivalent straight or branched chained saturated hydrocarbon radicals having from 1 to 6 carbon atoms. C₁₋₆ alkanediyl group, is preferably a C₁₋₄ alkanediyl group, a C₁₋₃ alkanediyl or more preferably a C₁₋₂ alkanediyl. Examples include, but are not limited to methanediyl, ethanediyl, propanediyl, butanenediyl, pentanediyl and hexanediyl. Preferred are methanediyl, ethanediyl and propanediyl.

As used herein, the term "C₂₋₇alkenediyl" as group or as part of a group defines bivalent straight or branched (carbon number limitation permitting) chained unsaturated hydrocarbon radicals having from 2 to 7 carbon atoms. Non limiting examples of C₂₋₇alkenediyl are: -C=CH-, - CH=C(CH₃)CH₂-, -CH=CH-CH₂-.

As used herein, the term "3-8 membered saturated ring" means saturated cyclic hydrocarbon (cycloalkyl) with 3, 4, 5, 6, 7 or 8 carbon atoms and is generic to cyclopropyl, cyclobutyl, cyclopentyl, cyclohexyl, cycloheptyl or cyclooctyl. Depending on the dimension of the ring, it can be also of bicyclic structure, such as a bicycle[3.1.0]hexane, bicycle[4.1.0]heptane, octahydropentalene and the like. In a particular embodiment of the invention, the 3-8 membered saturated ring" is restricted to a "3-7 membered saturated ring". Said saturated ring optionally contains one or more heteroatoms (also referred to as heterocyclyl or heterocyclic ring or heterocycloalkyl), such that at least one carbon atom is replaced by a heteroatom selected from N, O and S, in particular from N and O. Examples include, but are not limited to oxetanyl, azetidinyl, tetrahydro-2H-pyranyl, piperazinyl, piperidinyl, tetrahydrofuranyl, morpholinyl, thiomorpholinyl, thiazolidinyl, thiolane 1,1-dioxide, pyrrolidinyl, azepanyl, diazepanyl, oxazepanyl, thiazepanyl, azocanyl or oxazocanyl. Preferred are saturated cyclic hydrocarbons with 3 or 4 or 5 carbon atoms and 1 oxygen or 1 nitrogen atom. Examples include oxetanyl, tetrahydrofuranyl, tetrahydro-2H-pyranyl, piperidinyl or pyrrolidinyl. Preferred 3-7 membered saturated rings are oxetanyl, cyclopropyl and cyclobutyl.

As used herein, the expression "3-8 membered partially saturated ring" indicates a ring containing 3 to 8 carbon atoms and at least one double bond. Depending on the dimension of the ring, it can be of a cyclic or bicyclic structure. In a particular embodiment of the invention, the 3-8 membered partially saturated ring" is restricted to a "5-7 membered partially saturated ring". Each of the above rings may optionally contain one or more heteroatoms, such that at least one carbon is replaced by by a heteroatom selected from N, O and S, in particular from N and O. Examples include, but are not limited to cyclopentenyl, cyclohexenyl, cyclohexa-1,3-dienyl, cyclohexa-1,4-dienyl, cycloheptenyl, cyclohepta-1,4-dienyl, dihydrofuranyl, dihydropyrrole, dihydropyranyl, hexahydro-lH-cyclopenta[c]furanyl and the like.

It should be noted that different isomers of the various heterocycles may exist within the definitions as used throughout the specification. For example, pyrrolyl may be 1H-pyrrolyl or 2H-pyrrolyl.

It should also be noted that the radical positions on any molecular moiety used in the definitions may be anywhere on such moiety as long as it is chemically stable. For example, pyridyl includes 2-pyridyl, 3-pyridyl, 4-pyridyl.

As used herein, the term "halogen" refers to fluorine, chlorine, bromine and iodine, of which fluorine, chlorine and bromine are preferred.

The term "heteroatom" refers to an atom other than carbon or hydrogen in a ring structure or a saturated backbone as defined herein. Typical heteroatoms include N(H), O, S.

As used herein, the term "C₃₋₈ cycloalkyl" means saturated cyclic hydrocarbon (cycloalkyl) with 3 or 4, 5, 6, 7 or 8 carbon atoms and is generic to cyclopropyl, cyclobutyl, cyclopentyl, cyclohexyl, cycloheptyl or cyclooctyl.

The term "C₁₋₆alkylaryl" as used herein indicates one or more aryl groups appended to a C₁₋₆alkyl radical. As used herein, the term "C₁₋₆alkylheteroaryl" indicates one or more heteroaryl groups appended to a C₁₋₆alkyl radical. As used herein, the term "C₁₋₆alkyl-C₃₋₈cycloalkyl" indicates one or more C₃₋₈cycloalkyl groups appended to a C₁₋₆alkyl radical.

The terms "spiro-C₃₋₈cycloalkyl" or "spiro-C₃₋₈heterocycloalkyl" indicate respectively a C₃₋₈cycloalkyl or a C₃₋₈heterocycloalkyl forming a bicyclic organic compound with rings connected through just one atom. The rings can be different in nature or identical. The connecting atom is also called the spiroatom, most often a quaternary carbon ("spiro carbon").

Included in the instant invention is the free base of compounds of the invention as well as the pharmaceutically acceptable salts and stereoisomers thereof. Some of the specific compounds exemplified herein are the protonated salts of amine compounds. Compounds of the invention containing one or more N atoms may be protonated on any one, some or all of the N atoms. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of the disclosure. The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base. In a preferred embodiment, the compounds of the invention have at least one acidic proton and the corresponding sodium or potassium salt can be formed, for example, by reaction with the appropriate base.

Thus, pharmaceutically acceptable salts of the compounds of this invention include the conventional non-toxic salts of the compounds of this invention as formed by reacting a basic instant compound with an inorganic or organic acid or an acid compound with an inorganic or organic base. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Convential non-toxic salts further include those derived from an inorganic base, such as potassium, sodium hydroxide, magnesium or calcium hydroxide, as well as salts prepared from organic bases, such as ethylene diamine, lysine, tromethamine, meglumine and the like. Preferably, a pharmaceutically acceptable salt of this invention contains one equivalent of a compound of formula (I) and 1, 2 or 3 equivalent of an inorganic or organic acid or base. More particularly, pharmaceutically acceptable salts of this invention are the tartrate, trifluoroacetate or the chloride salts.

When the compound of the present invention is acidic, suitable "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamin, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

The compounds of the present invention find use in a variety of applications for human and animal health. The compounds of the present invention are inhibitors of hepatitis B virus (HBV). The compounds of the present invention are inhibitors of hepatitis B virus (HBV) useful for the treatment and/or prevention of HBV infection. In particular the compounds of the present invention are inhibitors of hepatitis B virus (HBV) core (HBc) protein useful for the treatment and/or prevention of a HBV infection.

The compounds, compositions and methods provided herein are particularly deemed useful for treating, ameliorating or preventing HBV infection and related conditions, including chronic hepatitis B, HBV/HDV co-infection, HBV/HCV co-infection, HBV/HIV co-infection, inflammation, necrosis, cirrhosis, hepatocellular carcinoma, hepatic decompensation and hepatic injury from an HBV infection.

In the present invention, the expression "HBV infection" comprises any and all conditions deriving from infection with HBV, including but not limited to hepatitis B, preferably chronic hepatitis B, HBV/HDV co-infection, HBV/HCV coinfection, HBV/HIV coinfection. Expressions like "treating, eradicating, reducing, slowing or inhibiting an HBV infection" are used to indicate the application or administration of a therapeutic agent, i.e., a compound of the invention (alone or in combination with another pharmaceutical agent), to a patient or application or administration of a therapeutic agent to an isolated tissue or cell line from a patient (e.g., for diagnosis or ex vivo applications), who has an HBV infection, a symptom of HBV infection or the potential to develop an HBV infection, with the purpose to cure, heal, alleviate, relieve, alter, remedy, ameliorate, improve or affect the HBV infection, the symptoms of HBV infection, or the potential to develop an HBV infection. Such treatments may be specifically tailored or modified, based on knowledge obtained from the field of pharmacogenomics.

Efficacy of treatment may be determined using quantification of viral load or other evidence of infection, such as through measurement of HBeAg, HBsAg, HBV DNA levels, ALT activity levels, serum HBV levels, and the like, thereby allowing adjustment of treatment dose, treatment frequency, and treatment length.

The compounds of the invention can reduce viral load in an individual suffering from an HBV infection. In a non limiting embodiment, the compounds of the invention result in viral load reduction during therapy in an individual in need thereof from a minimum of one- or two-log decrease to a maximum of about eight-log decrease.

As used herein, the expression "remission of hepatic injury from an HBV infection" means that the chronic necroinflammatory liver disease has been halted by the fact that the viral antigens have disappeared from the organ (and the immune system no longer attacks the liver cells).

As used herein, the term "prophylactically treating" means no disorder or disease development if none had occurred, or no further disorder or disease development if there had already been development of the disorder or disease. Also considered is the ability of one to prevent some or all of the symptoms associated with the disorder or disease. An example of prophylactic treatment might also indicate the necessity of reducing the risk of infecting a liver graft (in case of liver transplant in chronically infected patients) or infecting newborns (in case of chronically infected mothers that pass the virus at time of delivery).

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants. Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulstion.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS™ model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound(s) of the invention are employed. (For purposes of this application, topical application shall include mouth washes and gargles.)

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

The compounds of the invention may be presented in a liposome or other micro particulate or other nanoparticle designed to target the compound. Acceptable liposomes can be neutral, negatively, or positively charged, the charge being a function of the charge of the liposome components and pH of the liposome solution. Liposomes can be normally prepared using a mixture of Phospholipids and cholesterol. Suitable phospholipids include phosphatidylcholine, phosphatidylethanolamine, phosphatidic acid, phosphotidylglycerol, phosphatidylinositol. Polyethylene glycol can be added to improve the blood circulation time of liposomes. Acceptable nanoparticles include albumin nanoparticles and gold nanoparticles.

When a compound according to this invention is administered into a human subject, the daily dosage will normally be determined by the prescribing physician with the dosage generally varying according to the age, weight, sex and response of the individual patient, as well as the severity of the patient's symptoms.

In one exemplary application, a suitable amount of compound is administered to a mammal undergoing anti HBV treatment. Administration generally occurs in an amount between about: 0.01 mg/kg of body weight to about 100 mg/kg of body weight per day, preferably between about 0.01 mg/kg of body weight to about 60 mg/kg of body weight per day, preferably between about 0.1 mg/kg of body weight to about 50 mg/kg of body weight per day, preferably between about 0.5 mg/kg of body weight to about 40 mg/kg of body weight per day.

The instant compounds are also useful in combination with known therapeutic agents for simultaneous, separate or sequential administration.

In an embodiment, the compounds of the present invention may be used in combination with at least one or more additional therapeutic agents, in particular anti-HBV agents.

The indication that compounds of the invention are for use in the treatment and/or prevention of a HBV infection indicates that the compounds are efficacious for treating, eradicating, reducing, slowing or inhibiting an HBV infection.

The term "anti-HBV agent", or more simply "HBV antiviral(s)" also includes compounds that are therapeutic nucleic acids, antibodies or proteins either in their natural form or chemically modified and/or stabilized. The term therapeutic nucleic acid includes but is not limited to nucleotides and nucleosides, oligonucleotides, polynucleotides, of which non limiting examples are antisense oligonucleotides, miRNA, siRNA, shRNA, therapeutic vectors and DNA/RNA editing components.

The term anti-HBV agent also includes compounds capable of treating HBV infection via immunomodulation, i.e. immunomodulators or immunomodulating compounds. Examples of immunomodulators are interferon-a (IFN-α), pegylated interferon-a or stimulants of the innate immune system such as Toll-like receptor 7 and/or 8 agonists and therapeutic or prophylactic vaccines. One embodiment of the present invention relates to combinations of a compound of formula (I) or any subgroup thereof, as specified herein, with an immunomodulating compound, more specifically a Toll-like receptor 7 and/or 8 agonist.

The additional HBV antiviral(s) can be selected for example, from therapeutic vaccines; RNA interference therapeutic/antisense oligonucleotides (e.g. siRNA, ddRNA, shRNA); immunomodulators (such as TLR agonists (e.g. TLR7, TLR8 or TLR9 agonists); STING agonists; RIG-I modulators; NKT modulators; IL agonists; Interleukin or other immune active proteins, therapeutic and prophylactic vaccines and immune checkpoint modulators; HBV entry inhibitors; cccDNA modulators (such as for example direct cccDNA inhibitors, inhibtors of cccDNA formation or maintenance, cccDNA epigenetic modifiers, inhibitors of cccDNA transcription); inhibitors of HBV protein espression; agents targeting HBV RNA; capsid assembly inhibitors/modulators; core or X protein targeting agents; nucleotide analogues; nucleoside analogues; interferons or modified interferons; HBV antivirals of distinct or unknown mechanism; cyclophilin inhibitors; sAg release inhibitors; HBV polymerase inhibitors; dinucleotides; SMAC inhibitors; HDV targeting agents; viral maturation inhibitors; reverse transcriptase inhibitors and HBV RNA destabilizers and other small-molecule inhibitors of HBV protein expression.

In particular, the combination of previously known anti-HBV agents, such as interferon-α (IFN-α), pegylated interferon-α, 3TC, tenofovir, lamivudine, entecavir, telbivudine, and adefovir or a combination thereof, and a compound of formula (I) or any subgroup thereof can be used as a medicine in a combination therapy. Additional examples of further therapeutic agents that may be combined with the compounds of the present invention include: Zidovudine, Didanosine, Zalcitabine, Stavudine, Abacavir, ddA Emtricitabine, Apricitabine, Atevirapine, ribavirin, acyclovir, valacyclovir, famciclovir, ganciclovir, valganciclovir, cidofovir, Efavirenz, Nevirapine, Delavirdine and Etravirine.

Particular examples of such HBV antiviral(s) include, but are not limited to:
- RNA interference (RNAi) therapeutics: TKM-HBV (also known as ARB-1467), ARB-1740, ARC-520, ARC-521, BB-HB-331, REP-2139, ALN-HBV, ALN-PDL, LUNAR-HBV, GS3228836, and GS3389404;
- HBV entry inhibitors: Myrcludex B, IVIG-Tonrol, GC-1102;
- HBV capsid inhibitor/modulators, core or X protein targeting agents, direct cccDNA inhibitors, inhibitors of cccDNA formation or maintenance, or cccDNA epigenetic modifiers: BAY 41-4109, NVR 3-778, GLS-4, NZ-4 (also known as W28F), Y101, ARB-423, ARB-199, ARB-596, AB-506, JNJ-56136379, ASMB-101 (also known as AB-V102), ASMB-103, CHR-101, CC-31326, AT-130, RO7049389.
- HBV polymerase inhibitors: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir (in particular tenofovir disoproxil fumarate (Viread), tenofovir alafenamide fumarate (TAF)), tenofovir disoproxil orotate (also known as DA-2802), tenofovir disopropxil aspartate (also known as CKD-390), AGX-1009, and CMX157);
- HBV RNA destabilizers and other small-molecule inhibitors of HBV protein expression: RG7834, AB-452;
- cyclophilin inhibitors: OCB-030 (also known as NVP-018), SCY-635, SCY-575, and CPI-431-32;
- dinucleotides: SB9200;
- compounds of distinct or unknown mechanism, such as but not limited to AT-61 ((E)-N-(1 - chloro-3 -oxo-1 -phenyl-3 -(piperidin-1 -yl)prop-1 -en-2-yl)benzamide), AT 130 ((E)-N-(1-bromo-1-(2-methoxyphenyl)-3-oxo-3-(piperidin-1-yl)prop-1-en-2-yl)-4-nitrobenzamide), and similar analogs; REP-9AC (also known as REP-2055), REP-9AC' (also known as REP-2139), REP-2165 and HBV-0259;
- TLR agonists (TLR7, 8 and/or 9): RG7795 (also known as RO-6864018), GS-9620, SM360320 (9-benzyl-8-hydroxy-2-(2-methoxy-ethoxy)adenine) and AZD 8848 (methyl [3-({[3-(6-amino-2-butoxy-8-oxo-7,8-dihydro-9H-pyrin-9-yl)propyl][3-(4-morpholinyl)propyl]amino}methyl)phenyl]acetate); ARB- 1598;
- RIG-I modulators: SB-9200;
- SMAC inhibitor: Birinapant
- Immune Check Point inhibitors: BMS-936558 (Opdivo (nivolumab)), KEYTRUDA® (pembrolizumab);
- therapeutic vaccines: HBsAG-HBIG, HB-Vac, ABX203, NASVAC, GS-4774, GX- 110 (also known as HB-110E), CVI-HBV-002, RG7944 (also known as INO-1800), TG-1050, FP-02 (Hepsyn-B), AIC649, VGX-6200, KW-2, TomegaVax-HBV, ISA-204, NU-500, INX-102-00557 HBV MVA, PepTcell;
- IL agonists and immune acting proteins: INO-9112; recombinant IL12;
- interferons: interferon alpha (IFN-α), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta- la, peginterferon beta-la, interferon delta, interferon lambda (IFN-λ), peginterferon lambda-1, interferon omega, interferon tau, interferon gamma (IFN-γ), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA, interferon alpha biobetter; in particular, peginterferon alpha-2a, peginterferon alpha-2b, glycosylated interferon alpha-2b, peginterferon beta-la, and peginterferon lambda-1; more in particular, peginterferon alpha-2a;
- HDV targeting agent: Lonafamib.

The term "administration" and variants thereof (e.g., "administering" a compound) in reference to a compound of the invention means introducing the compound or a prodrug of the compound into the system of the animal in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

In some embodiments, pulsed administration is more effective than continuous treatment because total pulsed doses are often lower than would be expected from continuous administration of the same composition. Each pulse dose can be reduced and the total amount of drug administered over the course of treatment is minimized. Individual pulses can be delivered to the patient continuously over a period of several hours, such as about 2, 4, 6, 8, 10, 12, 14 or 16 hours, or several days, such as 2, 3, 4, 5, 6 or 7 days.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician. The present invention will be described by means of the following examples and biological data are presented.

### MATERIALS AND METHODS

### Chemistry

### General

Unless otherwise indicated, commercially available reagents and solvents (HPLC grade) were used without further purification.

Specifically, the following abbreviations may have been used in the descriptions of the experimental methods:
NMR: Nuclear Magnetic Resonance; ¹H: proton; MHz: Megahertz; Hz: Hertz; HPLC: High Performance Liquid Chromatography; LC-MS: Liquid Chromatography Mass Chromatography Spectrum; s: second(s); min: minute(s); h: hour(s); mg: milligram(s); g: gram(s); Ml: microliter(s); mL: millilitre(s); mmol: millimole(s); nm: nanometer(s) µM: micromolar; M: molarity or molar concentration; Rt: retention time in minutes; sat.aq.: saturated aqueous solution; MW: microwave; Boc: *tert*-butyloxycarbonyl protecting group; DCM: dichloromethane; DIAD: Diisopropyl azodicarboxylate; DMF: dimethylformamide; DIPEA: *N,N-*diisopropylethylamine; DMSO: dimethylsulfoxide; EtOH: ethanol; EtOAc: ethyl acetate; IPA: isopropylamine; LiHMDS: Lithium bis(trimehtylsilyl)amide; MeOH: methanol; MeCN: Acetonitrile; PE: Petroleum Ether; PMB: p-methoxybenzyl protecting group; PyBop: Benzotriazole-1-yl-oxy-tris-pyrrolidino-phosphonium hexafluorophosphate; TFA: trifluoroacetic acid; eq.: equivalent(s); RT: room temperature; TBDMS: *tert*-butyldimethylsilyl; TEA: triethylamine; THF: tetrahydrofuran; *p*TSA: *para-*toluene sulfonic acid; TBTU: 2-(1H-Benzotriazole-1-yl)-1,1,3,3-tetramethylaminium tetrafluoroborate. Except where indicated otherwise, all temperatures are expressed in °C (degrees centigrade) or K (Kelvin).

The ¹H-NMR spectra were acquired with an Avance II 300 MHz Bruker spectrometer. The chemical shifts are expressed in parts per million (ppm, δ units). The coupling constants are expressed in Hertz (Hz) and the splitting patterns are described as s (singlet), bs (broad signal), d (doublet), t (triplet), q (quartet), quint (quintet), m (multiplet).

The LC-MS analyses were performed by means of an UPLC Acquity Waters System equipped with the SQD spectrometer, single quadrupole mass detector, and a TUV detector, using column 1: ACQUITY UPLC BEH SHIELD, RP₁₈ (2.1x50mm, id=1.7 µm); column2: ACQUITY UPLC HSS T3, RP₁₈ (2.1x50mm, id=1.8 µm) and column3: ACQUITY UPLC BEH SHIELD, RP₁₈ (2.1x100mm, id=1.7 µm). Column temperature 40°C. Sample temperature 25°C. Phase A was composed by water (HiPerSolv Chromanorm Water VWR for HPLC-MS) + 0,05% Trifluoroacetic Acid; Phase B by CH₃CN (HiPerSolv Chromanorm Acetonitrile SuperGradient VWR, suitable for UPLC/UHPLC instruments) + 0,05% Trifluoroacetic Acid; flow rate: 0,5 mL/min; UV detection (DIODE array) 200 nm; ESI+ and ESI- detection in the 100-1000 m/z range.
Method 1: column 1, run time: 3 minutes, run gradient: 5%B to 100%B in 2.80 min + 100%B for 0.2 min, equilibration time: 0,8 min, ionization mode: ESI⁺.
Method 2: column 2, run time: 4 minutes, run gradient: 0%B to 45%B in 3.5 min + 45%B to 100%B in 0.05 min +100%B for 0.45 min, equilibration time: 0,8 min, ionization mode: ESI⁺. Method 3: column 3, run time: 6 minutes, run gradient: 5%B to 100%B in 5 min + 100%B for 1 min, equilibration time: 2 min.
Method 4: column 3, run time: 6 minutes, run gradient: 5%B to 50%B in 5 min + 50%B to 100%B in 0.2 min 100%B for 0.8 min, equilibration time: 2 min, ionization mode: ESI⁺.
Method 5: column 1, run time: 3 minutes, run gradient: 5%B to 100%B in 2.80 min + 100%B for 0.2 min, equilibration time: 0,8 min, ionization mode: ESI⁺.
Method 6: column 2, run time: 4 minutes. run gradient: 0%B to 45%B in 3.5 min + 45%B to 100%B in 0.05 min +100%B for 0.45 min. Equilibration time: 0,8 min, ionization mode: ESI⁺. Method 7: column 3, run time: 6 minutes, run gradient: 5%B to 100%B in 5 min + 100%B for 1 min, equilibration time: 2 min, ionization mode: ESI⁺.
Method 8: column 3, run time: 6 minutes, run gradient: 5%B to 50%B in 5 min + 50%B to 100%B in 0.2 min 100%B for 0.8 min, Equilibration time: 2 min, ionization mode: ESI⁺.
Method 9: column 1. run time: 4 minutes, column 1, run time: 4 minutes, run gradient:5%B to 100%B in 3.00 min + 100%B for 1 min, equilibration time: 0,8 min, ionization mode: ESI⁺. Method 10: column 1. run time: 4 minutes, run gradient: 5%B to 100%B in 3.00 min + 100%B for 1 min, equilibration time: 0,8 min, Ionization Mode: ESI⁻.
Method 11: column 1, run time: 3 minutes, run gradient: 40%B to 100%B in 2.80 min + 100%B for 0.2 min, equilibration time: 0,8 min. Ionization Mode: ESI⁺.
Method 12: column 3, run time: 6 minutes, run gradient: 25%B to 70%B in 5 min + 100%B for 1 min, equilibration time: 2 min, Flow: 0,5 mL/min, ionization mode: ESI⁺.
Method 13: column 2, run time: 4 minutes, run gradient: 0%B to 60%B in 3.5 min + 60%B to 100%B in 0.05 min +100%B for 0.45 min, equilibration time: 0,8 min, ionization mode: ESI⁺. Method 14: column 2, run time: 4 minutes, run gradient: 0%B to 30%B in 3.5 min + 30%B to 100%B in 0.05 min +100%B for 0.45 min, equilibration time: 0,8 min, ionization mode: ESI⁺.

### Synthesis

In the following paragraphs, the Descriptions 1 to 13 illustrate the preparation of intermediates used to make compounds of the invention and salts thereof. The Examples 1 to 9 illustrate the preparation of the compounds of the invention and salts thereof. Where the compounds have more than one chiral center, it is understood that they might exist as mixtures of diastereoisomers or as single isomers. Both racemic and chiral compounds are within the scope of the present invention. The indicated procedures are provided merely for assistance to the skilled chemist. The starting material may not necessarily have been prepared from the batch of the Description or the Example referred to.

Where not otherwise indicated, starting materials and/or intermediates were obtained from commercial sources or can be obtained through synthetic procedures known in the chemistry literature. The indication of the commercial source of certain compounds in the description of the experimental procedure, when provided, is only for easy reference to skilled chemist and should not be interpreted as the indication to use only that particular commercial compound.

### Description D1: Ethyl (3aR,6aR)-1-((R)-2-hydroxy-1-phenylethyl)tetrahydro-1H-pyrrolo [3,4-c] isoxazole-5(3H)-carboxylate

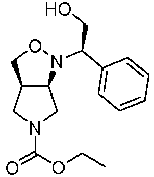

Title compound was prepared following the procedure reported in J. Org.Chem. 2003, 68, 8739-8741, starting from ethyl allyl(2-oxoethyl)carbamate (prepared as reported in US2018/0222918) and (R)-2-(hydroxyamino)-2-phenylethan-1-ol (prepared as reported in WO2010/016005). ¹H NMR (300 MHz, CHLOROFORM-d) δ ppm 1.15 (t, J=7.11 Hz, 3 H) 2.87 (dd, J=8.89, 4.40 Hz, 1 H) 3.06 - 3.56 (m, 5 H) 3.57 - 3.75 (m, 3 H) 3.75 - 3.83 (m, 1 H) 3.94 - 4.11 (m, 3 H) 4.27 (br t, J=8.12 Hz, 1 H) 7.22 - 7.36 (m, 5 H). Method 3; Rt=2.18min. m/z=307.32 (M+H)⁺.

### Description D2: Ethyl (3R,4R)-3-amino-4-(hydroxymethyl)pyrrolidine-1-carboxylate hydrochloride

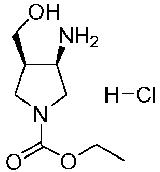

Compound **D1** (2.9g, 9.47mmol) was dissolved in methanol (150mL,3.703mol), palladium(II) hydroxide (3.06g, 4.35mmol) was added and the suspension was hydrogenated at 1atm at room temperature for 16hrs. Acetic acid (15.16mL, 265.05mmol) was added and the reaction stirred for 15min then filtered over paper, washing with methanol (approx 70mL). The solution was evaporated (30°C), the residue treated with 1M HCl (20mL) then further evaporated. The residue was dissolved in water (10mL), pH was adjusted with 1M HCl (3mL), washed with DCM and the acqueous layer was further evaporated and co-evaporated with toluene, in order to remove acetic acid traces, giving title compound **D2** (1.6g,7.12mmol) as off-white powder (Yield =75%). ¹H NMR (300 MHz, DMSO-d6 + TFA) δ ppm 1.18 (t, J=7.06 Hz, 3 H), 2.54 - 2.66 (m, 1 H), 3.17 - 3.66 (m, 6 H), 3.72 - 3.88 (m, 1 H), 3.93 - 4.14 (m, 2 H), 8.10 (br s, 3 H). Method 13; Rt=1.02min; m/z=189.15 (M+H)⁺.

### Description D3: methyl (R)-2-oxo-2-((1,1,1-trifluoropropan-2-yl)amino)acetate

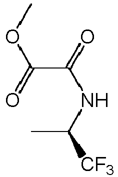

To a solution of (2R)-1,1,1-Trifluoro-2-propanamine hydrochloride (1:1) (500 mg, 3.34 mmol) (U23940, AurumPharmacuticals, CAS: 177469-12-4) and N-ethyl-N-isopropylpropan-2-amine (1.16 mL, 6.69 mmol) in DCM dry (3 mL, 0.047 mol), methyl 2-chloro-2-oxoacetate (0.31 mL, 3.34 mmol) was added dropwise at 0°C and under nitrogen atmosphere. The reaction was stirred at 0°C for 30min, then was quenched with ice and water. The organic phase was washed with IN HCl (3x 20mL) and brine. The organic phase was dried over Na₂SO₄ (anh.), then was filtered and concentrated to yield **D3** (567 mg, yield = 85%) as a colorless solid, that was used in the next synthetic step as such. Method 1: Rt=1.12 min, m/z=200.15 (M+H)⁺.

### Description D4: Ethyl (3aR,10aR)-8-((3-chloro-4-fluorophenyl)carbamoyl)-7-methyl-3a,4,10,10a-tetrahydro-1H,7H-dipyrrolo[3,4-b:3',4'-f][1,4,5]oxathiazocine-2(3H)-carboxylate 5,5-dioxide

The compound was prepared according to the following Scheme:

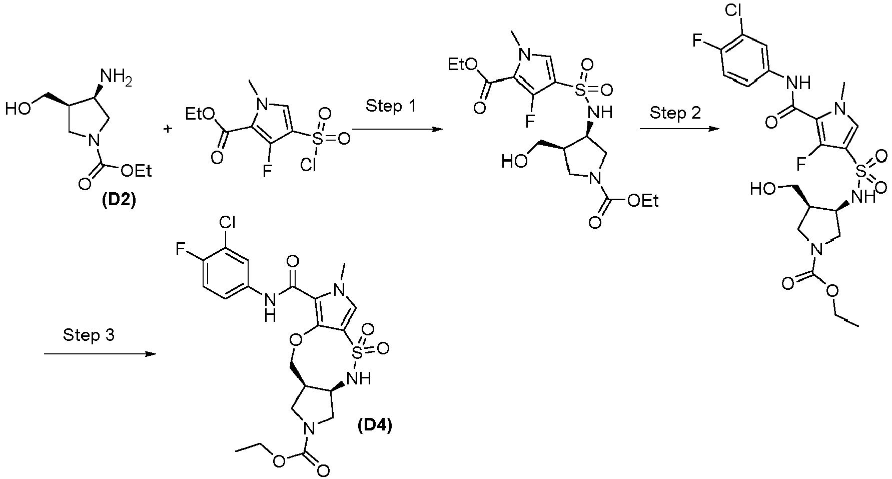

### Step 1

To a solution of **D2** (1351.61mg, 7.18mmol) in dry MeCN (24mL), DIPEA (2.5mL,14.36mmol) was added; then a solution of ethyl 4-(chlorosulfonyl)-3-fluoro-1-methyl-1H-pyrrole-2-carboxylate (1936.57mg,7.18mmol, prepared according to the procedure described in WO2017/001655) in dry MeCN (12mL) was added dropwise over 10 minutes. The reaction was stirred at RT for 90min then was concentrated under reduced pressure; diluted with EtOAc (130mL) and washed with 5%citric acid solution (40ml) and brine (20ml), dried over Na₂SO₄ (anh.), filtered and solvent removed under reduced pressure. The crude was purified by direct flash chromatography (eluent DCM/ AcOEt) to afford ethyl 4-(N-((3R,4R)-1-(ethoxycarbonyl)-4-(hydroxymethyl)pyrrolidin-3-yl)sulfamoyl)-3-fluoro-1-methyl-1H-pyrrole-2-carboxylate (2,9g, yield= 91%) as a white solid. Method 1: Rt= 1.44min; m/z=422.41 (M+H)⁺.

### Step 2

To a solution of ethyl 4-(N-((3R,4R)-1-(ethoxycarbonyl)-4-(hydroxymethyl)pyrrolidin-3-yl)sulfamoyl)-3-fluoro-1-methyl-1H-pyrrole-2-carboxylate (2.7 g, 6.43 mmol) prepared in Step 1 and 3-chloro-4-fluoroaniline (001682, Fluorochem, CAS: 367-21-5) in dry THF (50 mL), lithium bis(trimethylsilyl)amide (1M in THF) (3.33 mL, 20 mmol) was added dropwise at room temperature. After 60 min the reaction was quenched with water, diluted with DCM and washed with aq 5% citric acid and brine. The organic layer was dried over Na₂SO₄, filtered and concentrated under reduced pressure to afford ethyl (3R,4R)-3-((5-((3-chloro-4-fluorophenyl)carbamoyl)-4-fluoro-1-methyl-1H-pyrrole)-3-sulfonamido)-4-(hydroxymethyl)pyrrolidine-1-carboxylate as a brown foam that was used without further purification.

### Step 3

In a pressure vessel crude compound from previous step was dissolved in dry DMF (120 mL); cesium carbonate (3 equivalents) was added, the vial was sealed and mixture heated at 140°C with oil bath for 4h. The solvent was removed under reduced pressure, the residue was taken up with EtOAc and washed with water (x3). Organic layer was dried over Na2SO4 (anh.), filtered and solvent removed under reduced pressure. The resulting light-brown foam was then treated with Et₂O to remove residual solvent and obtain Ethyl (3aR,10aR)-8-((3-chloro-4-fluorophenyl)carbamoyl)-7-methyl-3a,4,10,10a-tetrahydro-1H,7H-dipyrrolo[3,4-b:3',4'-f][1,4,5]oxathiazocine-2(3H)-carboxylate 5,5-dioxide as light-brown solid, that was used in the next step without further purification. Method 1: Rt=2.09min; m/z = 501.30, 503.39 (M+H)⁺.

### Description D5: (3aR,10aR)-8-((3-chloro-4-fluorophenyl)carbamoyl)-7-methyl-2,3,3a,4,10,10a-hexahydro-1H,7H-dipyrrolo[3,4-b:3',4'-f][1,4,5]oxathiazocin-2-ium 5,5-dioxide iodide

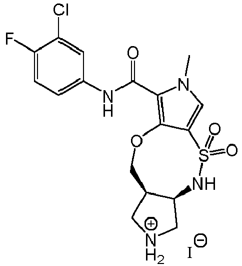

### Procedure 1

In a sealed vial **D4** (1 equivalent) was dissolved in dry DCM. Trimethylsilyl iodide (5 equivalents) was added and reaction mixture was heated at reflux (50°C) for 3h. Mixture was quenched by addition of methanol at 0°C, then evaporated under reduced pressure. The residue was triturated with Et₂O to obtain crude **D5** as orange solid, that was used in the next step without further purification. Method 1: Rt=1.44min; m/z = 429.30, 431.39 (M+H)⁺.

### Procedure 2

The compound was prepared according to the following Scheme:

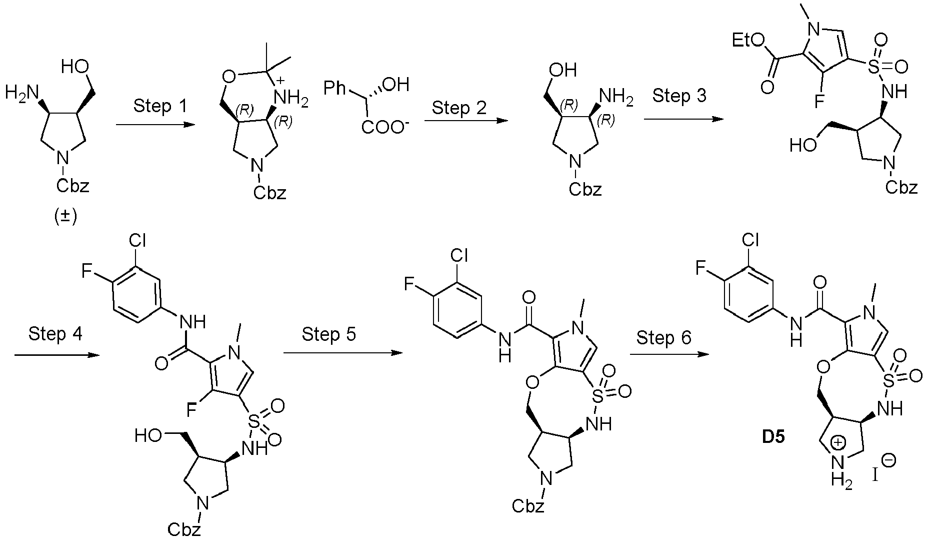

### Step 1

(±) Benzyl 3-amino-4-(hydroxymethyl)pyrrolidine-1-carboxylate was prepared following the procedure reported in J. Med.Chem. 2007, 50, 5493-5508 starting from 2,2-dimethoxyethan-1-amine (094452, Flurochem, CAS: 22483-09-6). ¹H NMR (300 MHz, DMSO-*d*₆ + TFA) δ ppm 2.55 - 2.68 (m, 1 H) 3.27 - 3.38 (m, 1 H) 3.43 - 3.67 (m, 5 H) 3.84 (br s, 1 H) 5.04 - 5.13 (m, 2 H) 7.29 - 7.41 (m, 5 H) 7.90 - 8.02 (br s, 2 H). Method 2; Rt=1.81min. m/z=251.25 (M+H)⁺.

The compound (11.52 g, 46.02 mmol) in acetone (16 mL) was treated with 2-methoxypropene (8.81 mL, 92.02 mmol) (174645, Sigma Aldrich, CAS: 116-11-0). The solution was stirred at room temperature for 1 h and subsequently concentrated under reduced pressure to remove the volatiles. The crude product (13.38g, 46.08.05 mmol) was taken in dry acetone (115 mL) and treated with (S)-2-hydroxy-2-phenylacetic acid (7.011g, 46.08 mmol) (046847, Fluorochem, CAS: 17199-29-0). Mixture was cooled to -5 °C and stirred for 12 h. The resulting white precipitate was filtered and washed 3 times with 60 mL of dry acetone, cooled at -5 °C, yielding benzyl (4aR,7aR)-2,2-dimethylhexahydropyrrolo[3,4-d][1,3]oxazine-6(4H)-carboxylate [(S)-mandelate] as white solid (6.2 g, y =30%).

### Step 2

Benzyl (4aR,7aR)-2,2-dimethylhexahydropyrrolo[3,4-d][1,3]oxazine-6(4H)-carboxylate [(S)-Mandelate] from step 1 (500 mg,1.13 mmol) was dissolved in 1 ml of absolute ethanol and H₂SO₄ (5% solution, 0.5 mL). Mixture was stirred for 3 h at room temperature. The resulting mixture was treated with 2M NaOH (1mL) solution and ethanol removed under reduced pressure. The aqueous residue was extracted with AcOEt (3x20ml). Benzyl (3R,4R)-3-amino-4-(hydroxymethyl)pyrrolidine-1-carboxylate (245 mg, y = 87%) was used in the next step without any further purification. Method 2; Rt=1.81min. m/z=251.25 (M+H)⁺

### Step 3

Ethyl 4-(chlorosulfonyl)-3-fluoro-1-methyl-1H-pyrrole-2-carboxylate (3.05g, 11.3mmol) was added portionwise to a suspension of benzyl (3R,4R)-3-amino-4-(hydroxymethyl)pyrrolidine-1-carboxylate (2.83g, 11.3mmol) and DIPEA (4mL, 22.96mmol) in MeCN (40ml). The yellow solution was stirred at RT overnight. The reaction was concentrated under reduced pressure, then was diluted with EtOAc (100mL), washed with aq 5% citric acid (x2) and s.s. NaHCO₃. The organic layer was dried over Na₂SO₄, filtered and concentrated under reduced pressure. The resulting crude was purified by flash chromatography on silica gel (eluent gradient from DCM/AcOEt: 5:5 to 100% EtoAc, to obtain ethyl 4-(N-((3R,4R)-1-((benzyloxy)carbonyl)-4-(hydroxymethyl)pyrrolidin-3-yl)sulfamoyl)-3-fluoro-1-methyl-1H-pyrrole-2-carboxylate (4.05g, y=74%) as off-white foam. ¹H NMR (300 MHz, DMSO-*d*₆+TFA) δ ppm 1.27 (q, *J*=6.60 Hz, 3 H) 2.31 - 2.43 (m, 1 H) 3.12 - 3.27 (m, 1 H) 3.27 - 3.48 (m, 4 H) 3.49 - 3.60 (m, 1 H) 3.67 - 3.94 (m, 4 H) 4.13 - 4.38 (m, 2 H) 4.92 - 5.16 (m, 2 H) 7.28 - 7.44 (m, 5 H) 7.56 (d, J=4.58 Hz, 1 H) 7.99 (br d, J=7.90 Hz, 1 H). Method 1; Rt=1.80min. m/z= 484.4 (M+H)⁺.

### Step 4

To a solution of ethyl 4-(N-((3R,4R)-1-((benzyloxy)carbonyl)-4-(hydroxymethyl)pyrrolidin-3-yl)sulfamoyl)-3-fluoro-1-methyl-1H-pyrrole-2-carboxylate (110.mg, 0.230mmol) and 3-chloro-4-fluoroaniline (0.03mL, 0.250mmol) in dry THF, (1.8mL), IN lithium bis(trimethylsilyl)amide in toluene (1.15mL, 1.15mmol) was added at RT for 1h. UPLC-MS analysis indicated complete conversion. The reaction was diluted with toluene, cooled at 0°C and quenched with 2M HCl aq, then was stirred for 10min at RT. The two phases were separated and the organic phase was washed with 2M HCl aq and sat. NaHCO₃, then was dried over Na₂SO₄, filtered and solvent removed under reduced pressure. The resulting crude material was used in the next step without any further purification. Method 1; Rt=2.11min. m/z= 583.29 (M+H)⁺.

### Step 5

To a solution of crude benzyl (3R,4R)-3-((5-((3-chloro-4-fluorophenyl)carbamoyl)-4-fluoro-1-methyl-1H-pyrrole)-3-sulfonamido)-4-(hydroxymethyl)pyrrolidine-1-carboxylate from Step 4 (142.92mg, 0.250mmol) in dry DMF (4mL), cesium carbonate (199.68mg, 0.610mmol) was added; the vial was sealed, the mixture heated to 135°C and stirred at the same temperature for 4h. EtOAc and water were added; the organic phase was washed again with water (x2), dried over Na₂SO₄, filtered and evaporated. The resulting crude was purified by flash chromatography on silica gel (eluent gradient from 100% DCM to DCM/EtOAc 70/30), to obtain desired compound. Method 1; Rt=2.30min. m/z= 563.30 (M+H)⁺.

### Step 6

Benzyl (3aR,10aR)-8-((3-chloro-4-fluorophenyl)carbamoyl)-7-methyl-3a,4,10,10a-tetrahydro-1H,7H-dipyrrolo[3,4-b:3',4'-f][1,4,5]oxathiazocine-2(3H)-carboxylate 5,5-dioxide from Step 5 (100mg, 0.180mmol) was dissolved in dry MeCN (2.5mL, 0.048mol). Trimethylsilyl iodide (80mL, 0.370mmo) was added and mixture was stirred at RT for 30min. Mixture was quenched by addition of methanol (1mL) at 0°C, stirred for 10min at the same temperature, then evaporated under reduced pressure. The resulting crude **D5** was used in the next step without further purification. Method 1: Rt=1.44min; m/z = 429.30(M+H)⁺.

### Description D6: (3aR,10aR)-8-((3,4-difluorophenyl)carbamoyl)-7-methyl-2,3,3a,4,10,10a-hexahydro-1H,7H-dipyrrolo[3,4-b:3',4'-f][1,4,5]oxathiazocin-2-ium 5,5-dioxide iodide

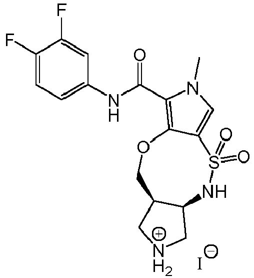

Prepared similarly as described for compound **D5,** using 3,4-difluoroaniline instead of 3-chloro-4-fluoroaniline to afford **D6** as dark-yellow solid, that was used in the next step without further purification. Method 1; Rt=1.34min. m/z= 413.36 (M+H)⁺.

### Description D7: (3aR,10aR)-8-((3-(difluoromethyl)-4-fluorophenyl)carbamoyl)-7-methyl-2,3,3a,4,10,10a-hexahydro-1H,7H-dipyrrolo[3,4-b:3',4'-f][1,4,5]oxathiazocin-2-ium 5,5-dioxide iodide

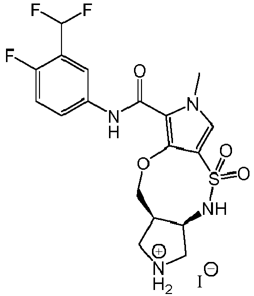

Prepared similarly as described for compound **D5,** using 3-(difluoromethyl)-4-fluoroaniline instead of 3-chloro-4-fluoroaniline to afford **D7** as a solid, that was used in the next step without further purification. Method 1; Rt=1.31min. m/z= 445.37 (M+H)⁺.

### Description D8: (3aR,10aR)-8-((4-fluoro-3-(trifluoromethyl)phenyl)carbamoyl)-7-methyl-2,3,3a,4,10,10a-hexahydro-1H,7H-dipyrrolo[3,4-b:3',4'-f][1,4,5]oxathiazocin-2-ium 5,5-dioxide iodide

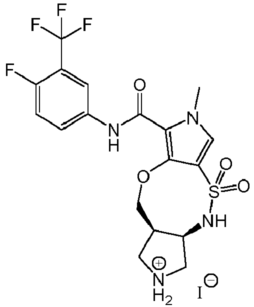

Prepared similarly as described for compound **D5,** using 4-Fluoro-3-(trifluoromethyl)aniline instead of 3-Chloro-4-fluoroaniline to afford **D8** as a solid, that was used in the next step without further purification. Method 1; Rt=1.51min. m/z= 463.41 (M+H)⁺.

### Description D9: (3aR,10aR)-8-((4-fluoro-3-methylphenyl)carbamoyl)-7-methyl-2,3,3a,4,10,10a-hexahydro-1H,7H-dipyrrolo[3,4-b:3',4'-f][1,4,5]oxathiazocin-2-ium 5,5-dioxide iodide

The compound was prepared according to the following Scheme:

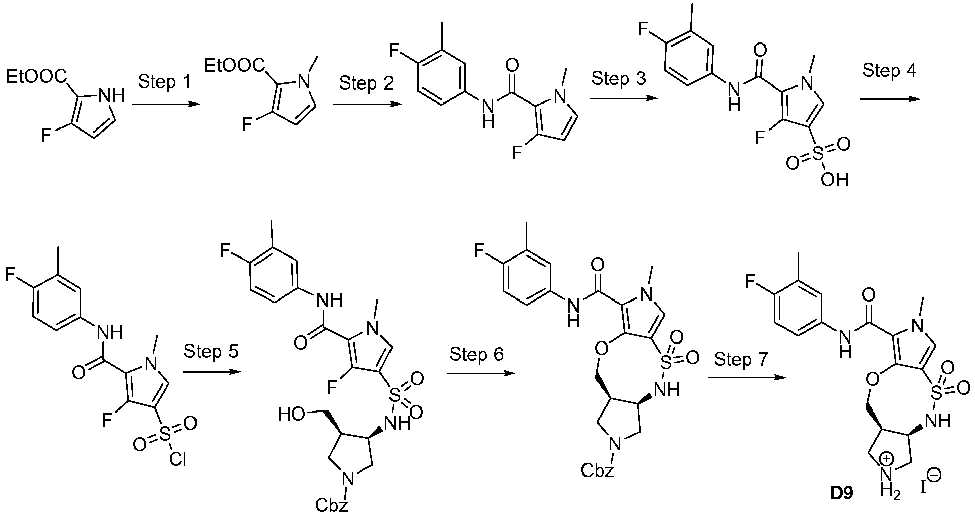

### Step 1

To a solution of ethyl 3-fluoro-1H-pyrrole-2-carboxylate (12.5 g, 79.6 mmol) (231254, Fluorochem, CAS: 168102-05-4) in dry DMF (125 mL) cooled to 0°C under nitrogen atmosphere, sodium hydride (60% weight in mineral oil, 3.7 g, 92.5 mmol) was added portion wise over 30 min. The reaction mixture was stirred for further 20 min then iodomethane (5.8 mL, 93.2 mmol) was added dropwise over 30 min. The mixture was stirred for further 30 min at the same temperature then quenched with 2N HCl (20 mL). The reaction mixture was dumped into water (120 mL) and toluene (650 mL) and the mixture was vigorously stirred for 10 min. The two phases were separated and the organic phase washed with water (250 mL) and brine (250 mL), dried over Na₂SO₄ (anh.) and filtered. Ethyl 3-fluoro-1-methyl-1H-pyrrole-2-carboxylate (13.6 g) was obtained as a pale yellow oil after solvent evaporation and used without further purification. ¹H NMR (300 MHz, DMSO-d6) δ ppm 1.27 (t, J=7.11 Hz, 3 H), 3.78 (s, 3 H), 4.23 (q, J=7.06 Hz, 2 H), 5.99 (d, J=3.03 Hz, 1 H), 7.00 (dd, J=5.27, 3.07 Hz, 1 H).

### Step 2

Ethyl 3-fluoro-1-methyl-1H-pyrrole-2-carboxylate (13.6 g, 79.5 mmol), prepared in Step 1, and 4-fluoro-3-methylaniline (10.3 g, 82.3 mmol) (006273, Fluorochem, CAS: 452-69-7) were dissolved in dry toluene (50 mL). LiHMDS (140 mL, 1 M in toluene, 140 mmol) was added dropwise over 30 min and the reaction mixture was stirred at room temperature for further 30 min. The reaction mixture was cooled at 0°C and slowly quenched with 2N HCl (200 mL), diluted with water (200 mL) and toluene (200 mL) and stirred at RT for 20 min. The two phases were separated and the organic phase washed with sat NaHCO3 (200 mL) and brine (200 mL), dried over Na₂SO₄ (anh.) and filtered. 3-fluoro-N-(4-fluoro-3-methylphenyl)-1-methyl-1H-pyrrole-2-carboxamide (19.8 g) was obtained as a light brown solid after solvent evaporation and used without further purification. ¹H NMR (300 MHz, DMSO-d6) δ ppm 2.22 (s, 3 H), 3.76 (s, 3 H), 6.01 (d, J=3.03 Hz, 1 H), 6.91 (dd, J=5.27, 3.07 Hz, 1 H), 7.08 (t, J=9.22 Hz, 1 H), 7.35 - 7.53 (m, 1 H), 7.59 (dd, J=7.06, 2.20 Hz, 1 H), 9.50 (br s, 1 H).

### Step 3

To a solution of 3-fluoro-N-(4-fluoro-3-methylphenyl)-1-methyl-1H-pyrrole-2-carboxamide (19.8 g, 79.5 mmol), prepared in Step 2, in dry DCM (90 mL) cooled to 0°C under nitrogen atmosphere, chlorosulfonic acid (5.7 mL, 85.6 mmol) dissolved in dry DCM (120 mL) was added dropwise over 90min. The reaction mixture was stirred at the same temperature for further 30 min; then the formed precipitate was filtered and washed several times with Et₂O. 4-fluoro-5-((4-fluoro-3-methylphenyl)carbamoyl)-1-methyl-1H-pyrrole-3-sulfonic acid (23.1 g, 88% yield over three steps) obtained as a light grey solid was dried under vacuum overnight and used without further purification. ¹H NMR (300 MHz, DMSO-d6) δ ppm 2.22 (s, 3 H), 3.70 (s, 3 H), 6.93 (d, J=5.04 Hz, 1 H), 7.07 (t, J=9.22 Hz, 1 H), 7.44 - 7.52 (m, 1 H), 7.60 (dd, J=7.06, 2.20 Hz, 1 H), 9.64 (s, 1 H).

### Step 4

Dry DMF (0.35 mL, 4.51mmol) was added to a suspension of 4-fluoro-5-((4-fluoro-3-methylphenyl)carbamoyl)-1-methyl-1H-pyrrole-3-sulfonic acid (14.9 g, 45.1 mmol), prepared in Step 3, in thionyl chloride (112 mL). The reaction mixture was heated to 75°C and stirred at the same temperature for 45min. The brown solution was cooled to RT, diluted with toluene (200 mL) and slowly poured into a mixture of toluene (200 mL) and ice (500 mL) under vigorous stirring. The biphasic system was stirred for 20min, the two phases were separated and the organic phase washed with ice-water (200 mL) and brine (200 mL), dried over Na₂SO₄ (anh.), filtered and concentrated under reduce pressure. The residue was purified on silica (eluent ETP/AcOEt gradient) yielding 4-fluoro-5 -((4-fluoro-3 -methylphenyl)carbamoyl)-1-methyl-1H-pyrrole-3 - sulfonyl chloride (13.9 g, 88% yield) as a beige powder. ¹H NMR (300 MHz, CDCl3) δ ppm 2.31 (s, 3 H), 4.06 (s, 3 H), 7.03 (t, J=8.89 Hz, 1 H), 7.26 - 7.36 (m, 2 H), 7.39 - 7.46 (m, 1 H), 7.72 (br d, J=8.16 Hz, 1 H).

### Step 5

Benzyl (3R,4R)-3-amino-4-(hydroxymethyl)pyrrolidine-1-carboxylate (245 mg, y = 87%) was prepared as described for the synthesis of **D5** (Procedure 2, Step 2). 4-Fluoro-5-((4-fluoro-3-methylphenyl)carbamoyl)-1-methyl-1H-pyrrole-3-sulfonyl chloride obtained in Step 4 as indicated above (302.36 mg, 0.870 mmol) was treated with benzyl (3R,4R)-3-amino-4-(hydroxymethyl)pyrrolidine-1-carboxylate (217 mg,0.870 mmol) in 5 ml of dry MeCN, and N-ethyl-N-isopropylpropan-2-amine (0.6 mL, 3.47mmol) was added. The resulting mixture was stirred at room temperature for 2.5 h. The reaction was diluted with EtOAc and washed with 5% citric acid solution and brine. Organic layer was dried over Na₂SO₄, filtered and solvent removed under reduced pressure. The resulting crude was purified by flash chromatography on silica gel (direct phase, eluent DCM/EtOAc) affording benzyl (3R,4R)-3-((4-fluoro-5-((4-fluoro-3-methylphenyl)carbamoyl)-1-methyl-1H-pyrrole)-3-sulfonamido)-4-(hydroxymethyl)pyrrolidine-1-carboxylate (420 mg, y = 86%) as white foam. ¹H NMR (300 MHz, DMSO-*d*₆+TFA) δ ppm 2.22 (s, 3 H) 2.30 - 2.43 (m, 1 H) 3.19 - 3.29 (m, 1 H) 3.29 - 3.51 (m, 4 H) 3.53 - 3.64 (m, 1 H) 3.70 - 3.92 (m, 4 H) 5.04 (d, *J*=4.22 Hz, 2 H) 7.10 (t, *J*=9.26 Hz, 1 H) 7.21 - 7.41 (m, 5 H) 7.43 - 7.54 (m, 2 H) 7.59 (br d, J=7.00 Hz, 1 H) 7.97 (d, *J*=6.88 Hz, 1 H) 9.99 (s, 1 H). Method 4; Rt=2.04min. m/z= 563 (M+H)⁺.

The enantiomeric ratio (ee>99%) of the title compound was determined by means of chiral HPLC (HPCL conditions: DIACEL CHIRALPACK IG COLUMN; eluents: Phase A: H₂O ultragrade 0.05 % TFA, MeCN ultragrade 0.05% TFA; flow rate, 1.0 ml/min, UV, 270 nM); retention time for (S,S), 30 min; and retention time for (R,R), 45min.

### Step 6

To a solution of compound coming from Step 5 (415 mg, 0.740 mmol) in dry DMF (2 ml), in sealed vial, cesium carbonate (604.58 mg, 1.84 mmol) was added; the resulting mixture was heated to 135 °C and stirred for 4 h. Water (10 mL) and toluene (40 mL) were added and the mixture was vigorously stirred for 5 min, the organic phase was collected, washed with water (20mL) and brine (20 mL), dried over Na₂SO₄ and evaporated. The resulting crude benzyl (3aR,10aR)-8-((4-fluoro-3-methylphenyl)carbamoyl)-7-methyl-3a,4,10,10a-tetrahydro-1H,7H-dipyrrolo[3,4-b:3',4'-f][1,4,5]oxathiazocine-2(3H)-carboxylate 5,5-dioxide (397mg, crispy off-white solid) was used without further purification. ¹H NMR (300 MHz, DMSO-*d*₆) δ ppm 2.24 (s, 3 H) 2.87 - 3.22 (m, 2 H) 3.37 - 3.57 (m, 2 H) 3.66 - 3.86 (m, 4 H) 3.86 - 4.02 (m, 1 H) 4.36 - 4.52 (m, 1 H) 4.52 - 4.66 (m, 1 H) 4.98 - 5.20 (m, 2 H) 7.11 (t, *J*=9.45 Hz, 1 H) 7.29 - 7.42 (m, 5 H) 7.43 - 7.54 (m, 2 H) 7.54 - 7.66 (m, 1 H) 8.41 (br s, 1 H) 9.33 (s, 1 H). Method 4; Rt=2.22 min. m/z= 543.24 (M+H)⁺.

### Step 7

To a solution of compound coming from Step 6 (960 mg, 1.77 mmol) was dissolved in dry MeCN (12 mL, 0.230 mol). Trimethylsilyl iodide (0.53 mL, 3.72 mmol) was added and the reaction was stirred at room temperature for 30 min. Then the mixture was treated by addition of methanol (1.2 mL) at 0 °C, stirred for 10 min at the same temperature, and then evaporated under reduced pressure. The residue was taken up with Et₂O/DCM 5:1 mixture (17 mL), then was filtered and solid washed many times with Et₂O, to obtain crude **D9** (833mg, y=87.8%) as light-yellow solid, that was used in the next step without further purification. Method 4; Rt=1.35min. m/z=409.24.25 (M+H)⁺.

### Description D10: methyl 2-((3aR,10aR)-8-((4-fluoro-3-methylphenyl)carbamoyl)-7-methyl-5,5-dioxido-3a,4,10,10a-tetrahydro-1H,7H-dipyrrolo[3,4-b:3',4'-f][1,4,5]oxathiazocin-2(3H)-yl)-2-oxoacetate

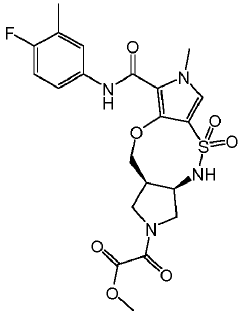

To a solution of **D9** (500 mg, 0.930 mmol) in dry MeCN (10 mL), N-ethyl-N-isopropylpropan-2-amine (0.229 mL, 1.28 mmol) was added. The solution was cooled at 0°C and methyl 2-chloro-2-oxoacetate (0.930 mmol, 0.344 mL) (151440, Sigma Aldrich, CAS: 5781-53-3), previously dissolved in dry MeCN (1 mL), was added dropwise. The reaction was stirred at the same temperature for 15 min and then was quenched by addition of 5% citric acid (1 mL), diluted with DCM and water. The organic phase was additionally washed with IN HCl and brine. The organic phase was dried over Na₂SO₄, filtered and concentrated under reduced pressure to give a yellow oil. The crude was stripped with DCM and finally with Pethroleum Ether to afford **D10** (390mg, y= 85%) as a yellow solid that was used in next step without purification. ¹H NMR (300 MHz, DMSO-d6) δ 2.24 (s, 3H), 2.98-3.12 (m, 1H), 3.13-4.12 (m, 11H), 4.44-4.68 (m, 2H), 7.11 (t, J=9.08 Hz, 1H), 7.44-7.54 (m, 2H), 7.55-7.63 (m, 1H), 8.47 (br d, J=9.72 Hz, 1H), 9.35 (s, 1H). Method 1: Rt=1.85 min, m/z=495 (M+H)⁺.

### Description D11: 2-((3aR,10aR)-8-((4-fluoro-3-methylphenyl)carbamoyl)-7-methyl-5,5-dioxido-3a,4,10,10a-tetrahydro-1H,7H-dipyrrolo[3,4-b:3',4'-f][1,4,5]oxathiazocin-2(3H)-yl)-2-oxoacetic acid

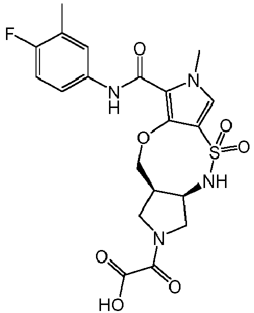

To a solution of **D10** (337 mg,0.680 mmol) in dry THF (1 mL,0.012 mol), a previously prepared solution of sodium hydroxide (81.78 mg, 2.04 mmol) in water (1 mL,0.056 mol), was added dropwise at room tempreture. The reaction was monitored after 5 min by UPLC/MS. Complete conversion was observed. The solution was cooled at 0 °C and quenched by adding HCl 4M till the formation of a white precipitate. The reaction was diluted with AcOEt and the two phases separated. The organic phase was washed with brine, dried over Na₂SO₄ (anh.), filtered and concentrated to afford **D11** (320mg, yield= 97.7%) as white solid that was used in the next synthetic step without further purification.. ¹H NMR (300 MHz, DMSO-d6) δ 2.24 (s, 3H), 2.96-3.10 (m, 1H), 3.11-4.11 (m, 8H), 4.43-4.69 (m, 2H), 7.11 (t, J=9.22 Hz, 1H), 7.43-7.53 (m, 2H), 7.54-7.63 (m, 1H), 8.48 (dd, J=9.81, 1.65 Hz, 1H), 9.35 (s, 1H), 14.06 (br s, 1H). Method 1: Rt=1.61 min, m/z=481.24 (M+H)⁺.

### Description D12: (3aR,10aR)-N-(4-fluoro-3-methylphenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide

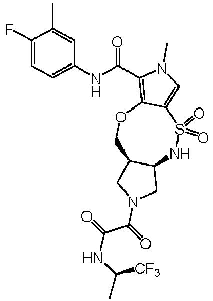

To a solution of **D11** (30 mg, 0.060 mmol), benzotriazol-1-yloxytris(dimethylamino)phosphonium hexafluorophosphate (30.38 mg, 0.070 mmol), DIPEA (25 uL, 0.140 mmol) in dry DMF (2.5 mL, 0.032 mol), (2R)-1,1,1-trifluoro-2-propanamine hydrochloride (Fluorochem, cat n° 093835) (0.190 mmol) was added at room temperature and reaction mixture stirred in the same conditions for 4 hrs. The reaction mixture was diluted with EtOAc (25 mL) and a mixture of 20 ml of water + 1ml of IN HCl. After phase separation the organic layers were washed with brine. The organic portion was dried over Na₂SO₄ (anh.), filtered and concentrated under reduced pressure. The crude was purified by Fraction-Lynx (H₂O/CH₃CN+1‰HCOOH) to afford **D12.** ¹H NMR (300 MHz, DMSO-d6) δ ppm 1.31 (t, J=6.88 Hz, 3 H), 2.24 (s, 3 H), 2.92 - 3.13 (m, 1 H), 3.15 - 3.68 (m, 2 H), 3.78 - 4.09 (m, 6 H), 4.43 - 4.56 (m, 1 H), 4.55 - 4.73 (m, 2 H), 7.11 (t, J=9.17 Hz, 1 H), 7.43 - 7.55 (m, 2 H), 7.54 - 7.64 (m, 1 H), 8.37 - 8.53 (m, 1 H), 9.21 - 9.31 (m, 1 H), 9.31 - 9.39 (m, 1 H). Method 3: Rt = 3.48 min; m/z = 576.33 (M+H)⁺.

### Example E1: (3aR,10aR)-N-(3-chloro-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide (E1)

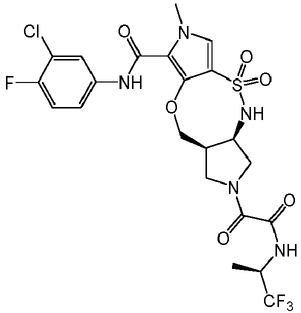

To a solution of crude **D5** (71.68mg, 0.130mmol) and **D3** (33.2mg, 0.160mmol) in ethanol (2mL), 2,3,4,6,7,8,9,10-octahydropyrimido[1,2-a]azepine (0.03mL,0.190mmol) was added and the resulting yellow solution was stirred at RT for 1h. Solvent was removed under reduced pressure, and the resulting crude was purified by preparative HPLC (H₂O/CH₃CN+0.1%HCOOH) to afford **E1.** ¹H NMR (300 MHz, DMSO-*d6*) δ ppm 1.31 (t, *J*=6.97 Hz, 3 H) 2.93 - 3.13 (m, 1 H) 3.20 (br t, *J*=11.46 Hz, 1 H) 3.36 - 3.67 (m, 2 H) 3.81 - 4.09 (m, 5 H) 4.44 - 4.70 (m, 3 H) 7.41 (t, *J*=9.03 Hz, 1 H) 7.50 (s, 1 H) 7.61 - 7.69 (m, 1 H) 7.99 (ddd, *J* =6.79, 4.03, 2.66 Hz, 1 H) 8.49 (br d, *J* =8.80 Hz, 1 H) 9.28 (br d, *J* =7.43 Hz, 1 H) 9.57 (d, *J* = 10.18 Hz, 1 H). Method 3; Rt= 3.67 min. m/z= 596.39 (M+H)⁺.

### Example E2: (3aR,10aR)-N-(3,4-difluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide (E2)

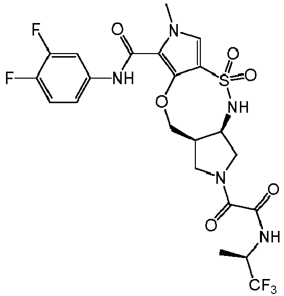

Prepared similarly as described for compound **E1** starting from **D6** instead of **D5**. The crude was purified by preparative HPLC (H₂O/CH₃CN+0.1%HCOOH) to afford **E2.** ¹H NMR (300 MHz, DMSO-*d*6+TFA) δ ppm 1.30 (t, *J*=6.97 Hz, 3 H) 2.86 - 3.12 (m, 1 H) 3.14 - 3.68 (m, 2 H) 3.81 (s, 3 H) 3.82 - 4.12 (m, 3 H) 4.42 - 4.55 (m, 1 H) 4.57 - 4.72 (m, 2 H) 7.30 - 7.47 (m, 2 H) 7.48 (s, 1 H) 7.75 - 7.95 (m, 1 H) 8.35 - 8.56 (m, 1 H) 9.16 - 9.33 (m, 1 H) 9.56 (d, *J*=9.72 Hz, 1 H). Method 3; Rt=3.51min. m/z=580.29 (M+H)+.

### Example E3: (3aR,10aR)-N-(3-(difluoromethyl)-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide (E3)

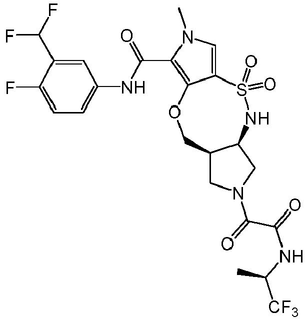

Prepared similarly as described for compound **E1** starting from **D7** instead of **D5**. The crude was purified by preparative HPLC (H₂O/CH₃CN+0.1%HCOOH) to afford **E3.** ¹H NMR (300 MHz, DMSO-*d*6) δ ppm 1.30 (t, *J* = 7.06 Hz, 3 H) 2.90 - 3.13 (m, 1 H) 3.15 - 3.70 (m, 2 H) 3.78 - 4.09 (m, 6 H) 4.44 - 4.68 (m, 3 H) 6.97 - 7.40 (m, 2 H) 7.47 (s, 1 H) 7.79 (br dd, *J* =7.70, 3.58 Hz, 1 H) 8.02 - 8.09 (m, 1 H) 8.45 (dd, *J* = 16.87, 9.90 Hz, 1 H) 9.18 - 9.30 (m, 1 H) 9.60 (d, *J*=9.63 Hz, 1 H). Method 3; Rt=3.51min. m/z=612.39 (M+H)+.

### Example E4: (3aR,10aR)-N-(4-fluoro-3-(trifluoromethyl)phenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide (E4)

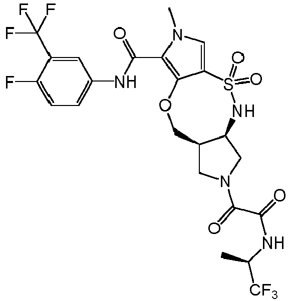

Prepared similarly as described for compound **E1** starting from **D8** instead of **D5**. The crude was purified by preparative HPLC (H₂O/CH₃CN+0.1%HCOOH) to afford **E4.** ¹H NMR (300 MHz, DMSO-*d*6+TFA) δ ppm 1.30 (t, *J*=7.11 Hz, 3 H) 2.88 - 3.14 (m, 1 H) 3.14 - 3.70 (m, 2 H) 3.81 (s, 3 H) 3.82 - 4.11 (m, 3 H) 4.43 - 4.55 (m, 1 H) 4.56 - 4.73 (m, 2 H) 7.36 - 7.62 (m, 2 H) 7.87 - 8.00 (m, 1 H) 8.14 - 8.25 (m, 1 H) 8.35 - 8.56 (m, 1 H) 9.17 - 9.32 (m, 1 H) 9.71 (d, *J*=9.45 Hz, 1 H). Method 3; Rt=3.75min. m/z=630.33 (M+H)+.

### Example E5: (3aR,10aR)-6-chloro-N-(3-chloro-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide (E5)

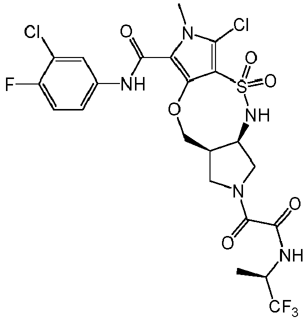

To a solution of **E1** (15mg,0.030mmol) in DCM (2.1mL), cooled to 0°C, sulfuryl dichloride (2.3µL,0.030mmol) previously dissolved in DCM (0.4mL), was added porionwise over 1min at 0°C. The reaction was left to RT and stirred for 3h. UPLC/MS indicated less than 50% conversion. Further sulfuryl dichloride (2 µL) dissolved in DCM (0.4mL) was added and the reaction was stirred at RT for further 2hrs. The reaction was quenched with water (0.5mL), then was concentrated under reduced pressure and was directly purified by preparative HPLC (H₂O/CH₃CN+0.1%HCOOH) to afford **E5.** ¹H NMR (300 MHz, DMSO-*d*₆) δ ppm 1.24 - 1.40 (m, 3 H) 2.84 - 3.06 (m, 1 H) 3.17 - 3.70 (m, 2 H) 3.73 - 4.16 (m, 6 H) 4.26 - 4.50 (m, 2 H) 4.52 - 4.74 (m, 1 H) 7.42 (s, 1 H) 7.58 - 7.74 (m, 1 H) 7.88 - 8.10 (m, 1 H) 8.48 - 8.92 (m, 1 H) 9.11 - 9.54 (m, 1 H) 9.71 - 9.99 (m, 1 H). Method 3; Rt: 3.82. m/z: 630.63 (M+H)⁺.

### Example E6: (3aR,10aR)-6-chloro-N-(3,4-difluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide (E6)

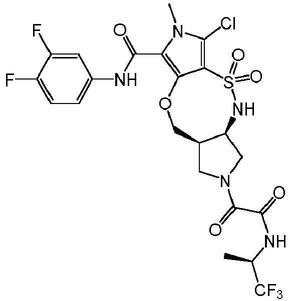

Prepared similarly as described for compound **E5** starting from **E2.** The crude was purified by preparative HPLC (H₂O/CH₃CN+0.1%HCOOH) to afford **E6.** ¹H NMR (300 MHz, DMSO-*d*6) δ ppm 1.26 - 1.38 (m, 3 H) 2.81 - 3.05 (m, 1 H) 3.33 (m, 2 H) 3.71 - 4.14 (m, 6 H) 4.33 - 4.51 (m, 2 H) 4.52 - 4.76 (m, 1 H) 7.27 - 7.58 (m, 2 H) 7.68 - 7.96 (m, 1 H) 8.32 - 8.91 (m, 1 H) 9.27 (br s, 1 H) 9.82 (d, J=7.70 Hz, 1 H). Method 3; Rt: 3.67. m/z: 614.42 (M+H)⁺.

### Example E7: (3aR,10aR)-6-chloro-N-(3-(difluoromethyl)-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide (E7)

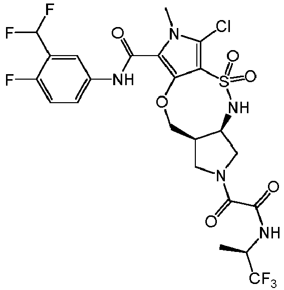

Prepared similarly as described for compound **E5** starting from **E3.** The crude was purified by preparative HPLC (H₂O/CH₃CN+0.1%HCOOH) to afford **E7.** ¹H NMR (300 MHz, DMSO-*d*₆+TFA) δ ppm 1.30 (t, J=7.20 Hz, 3 H) 2.79 - 3.08 (m, 1 H) 3.14 - 3.72 (m, 2 H) 3.72 - 4.17 (m, 6 H) 4.34 - 4.51 (m, 2 H) 4.51 - 4.75 (m, 1 H) 6.91 - 7.49 (m, 2 H) 7.80 (br dd, *J* =7.93, 3.81 Hz, 1 H) 8.03 (br s, 1 H) 8.67 (dd, *J*=18.75, 9.86 Hz, 1 H) 9.24 (t, *J* =8.53 Hz, 1 H) 9.86 (d, *J* =8.25 Hz, 1 H). Method 3; Rt: 3.66. m/z: 646.43 (M+H)⁺.

### Example E8: (3aR,10aR)-6-chloro-N-(4-fluoro-3-(trifluoromethyl)phenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:34,4'-f][1,4,5]oxathiazocine-8-carboxamide 5,5-dioxide (E8)

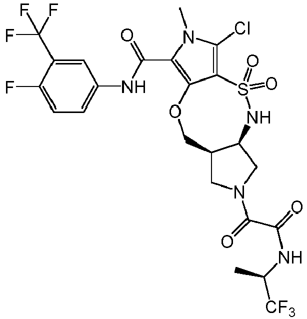

Prepared similarly as described for compound **E5** starting from **E4.** The crude was purified by preparative HPLC (H₂O/CH₃CN+0.1%HCOOH) to afford **E8.** ¹H NMR (300 MHz, DMSO-*d*₆) δ ppm 1.22 - 1.40 (m, 3 H) 2.85 - 3.03 (m, 1 H) 3.17 - 3.67 (m, 2 H) 3.69 - 4.13 (m, 6 H) 4.34 - 4.53 (m, 2 H) 4.53 - 4.75 (m, 1 H) 7.49 - 7.57 (m, 1 H) 7.89 - 8.03 (m, 1 H) 8.13 - 8.26 (m, 1 H) 8.49 - 8.88 (m, 1 H) 9.19 - 9.41 (m, 1 H) 9.91 - 10.06 (m, 1 H). Method 3; Rt: 3.89. m/z: 664.46 (M+H)⁺.

### Example E9: (3aR,10aR)-6-chloro-N-(4-fluoro-3-methylphenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide (E9)

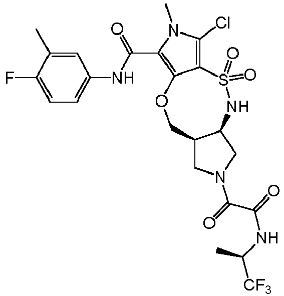

Prepared similarly as described for compound **E5** starting from **D12.** The crude was purified by preparative HPLC (H₂O/CH₃CN+0.1%HCOOH) to afford **E9.** ¹H NMR (300 MHz, DMSO-*d*₆) δ ppm 1.36 - 1.48 (m, 3 H) 2.33 - 2.38 (m, 3 H) 2.94 - 3.15 (m, 1 H) 3.29 - 3.77 (m, 2 H) 3.87 - 4.19 (m, 6 H) 4.47 - 4.62 (m, 2 H) 4.64 - 4.85 (m, 1 H) 7.23 (t, *J*=9.17 Hz, 1 H) 7.57 - 7.72 (m, 2 H) 8.67 (s, 1 H) 9.32 - 9.48 (m, 1 H) 9.65 - 9.77 (m, 1 H). Method 3; Rt: 3.69. m/z: 610.44 (M+H)⁺

### Biology

### Assay

### Cells and culture conditions

HepAD38 cell line (Ladner et al., Antimicrob Agents Chemother, 1997, 41, 1715-20) was used for HBV inhibition assays. HepAD38 is a subclone, derived from Hepatoblastoma cell line HepG2 (ATCC® Number: HB-8065™), that expresses HBV genome under the transcriptional control of a tetracycline-responsive promoter in a TET-OFF system: addition of tetracycline (TET) or doxycycline suppresses HBV replication, while its removal switches on the process allowing HBV viral particles release in the cell supernatant. HepAD38 cell line is maintained in DMEM/F12, supplemented with 10% of fetal bovine serum, 1% of glutamine, 1% of penicillin/streptomycin, 0.4 mg/ml G418 and 0,3 ug/ml tetracycline. For the HBV inhibition assay, doxycycline-free medium is used in order to allow virion production.

### Anti-HBV activity in vitro

HBV inhibition activity in vitro was performed in 96 multiwell plates. During the initial (primary) screening, compounds were first tested in triplicates at concentrations of 0.02 µM, 0.1µM, 0.5µM and 1µM. For selected compounds, an 8-point dose-response curve was obtained using 1:2 serial dilutions (starting from 0.01µM, 0.1µM, 0.4µM or 5 µM, depending on the degree of inhibition observed during the primary screening). From the dose-response curves, half maximal effective concentration (EC₅₀) could be calculated (see also below).

In more detail, compounds - typically dissolved in DMSO stock solutions - were diluted to 2x the final desired concentration in 100 µl of the above medium (without doxycycline) and plated in three replicates in the 96-well plates.

Simultaneously, HepAD38 cells - extensively pre-washed in tetracycline-free medium in order to induce HBV production - were suspended at 2^{∗}10⁴ cells in 100 µl of tetracycline-free medium and added to each well of the plate, to yield a final assay volume of 200 µl DMSO - used for stock solutions and compounds dilutions - which was always present in the assays at a final concentration of 0.5%.

Plates were then incubated 96 hours at 37°C and then subjected to cell viability assays and extracellular HBV quantification, in order to evaluate both the cytotoxic potential and the antiviral activity of compounds. Cytotoxicity was assessed by a commercial fluorescence assay that measures the metabolic activity of cells, directly related to cell viability (Cell Titer Blue, Promega). Anti-HBV activity was evaluated by quantification of extracellular HBV DNA with direct qPCR. In particular, supernatant was collected and centrifuged for cell debris clarification, viral DNA was extracted from virions by addition of lysis buffer (1 mM 1,4-dithiothreitol, 0.2% sodium dodecyl sulphate) and incubated at 95°C for 10 min. Samples were then diluted 1:40 and real time PCR amplification was performed with SYBR green assay (Power SYBR™ Green PCR Master Mix-Thermo Fisher Scientific) and specific HBV primer (HBV-DF:5'-ATTTGTTCAGTGGTTCGTAGGG-3' (SEQ ID No. 1), HBV-DR:5'-CGGTAAAAAGGGACTCAAGATG-3' (SEQ ID No. 2)).

Antiviral activity data for each compound are reported as EC₅₀ threshold value (see Table 1 legend). Excel and Graphpad Prism programs are typically used for data elaboration and EC₅₀ calculation.

### RESULTS

The exemplified compounds described herein were tested in the assays described above. All the compounds displayed no significant cytotoxicity at all concentrations of the dose-response curve (maximum tested dose of 0.01µM, 0.1µM, 0.4µM or 5 µM, depending on the compound potency). Results for HBV inhibition are reported in the following Table 1.

Legend: A = EC₅₀ less than 0.5 µM; B = EC₅₀ greater than 0.5 µM.

**Table 1**

| Example | Compound Name | **HBV inh EC₅₀ (µM)** |
|---|---|---|
| E1 | (3aR,10aR)-N-(3-chloro-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide | A |
| E2 | (3aR,10aR)-N-(3,4-difluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide | A |
| E3 | (3aR,10aR)-N-(3-(difluoromethyl)-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide | A |
| E4 | (3aR,10aR)-N-(4-fluoro-3-(trifluoromethyl)phenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide | A |
| E5 | (3aR,10aR)-6-chloro-N-(3-chloro-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide | A |
| E6 | (3aR,10aR)-6-chloro-N-(3,4-difluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide | A |
| E7 | (3aR,10aR)-6-chloro-N-(3-(difluoromethyl)-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide | A |
| E8 | (3aR,10aR)-6-chloro-N-(4-fluoro-3-(trifluoromethyl)phenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide | A |
| E9 | (3aR,10aR)-6-chloro-N-(4-fluoro-3-methylphenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide | A |

## Claims

1. A compound of general formula (I): 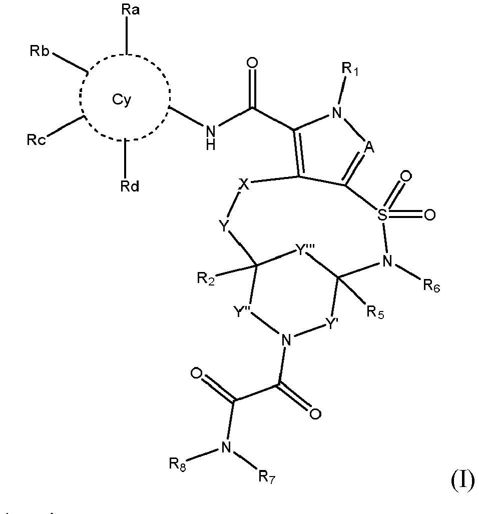 wherein:
Cy is aryl or heteroaryl;
A is C-R₃ or N;
X is O, S, NH, SO, SO₂ or a single bond;
Y, Y', Y" and Y'" are each independently C₁-₆alkanediyl or C₂₋₇alkenediyl, each optionally substituted with one or more R₄, or a single bond;
R₁ is H or C₁₋₆alkyl;
R₂ is selected from H, OH and C₁₋₆alkyl;
R₃ is selected from H, C₁₋₆alkyl, C₃₋₈cycloalkyl, haloC₁₋₆alkyl and halogen;
R₄ is selected from H, OH, C₁₋₆alkyl, C₃₋₈cycloalkyl and halogen or two geminal R₄ form together with the atom to which they are attached a spiro-C₃₋₈cycloalkyl or a spiro-C₃₋₈heterocycloalkyl;
R₅ is H or C₁₋₆alkyl;
or R₂ and R₅ taken together form a C₁₋₆alkanediyl bridge;
R₆ is selected from H, C₁₋₆alkyl, C₃₋₈cycloalkyl, C₃₋₈heterocycloalkyl, C₁₋₆alkylaryl, C₁₋₆alkylheteroaryl and C₁₋₆alkyl-C₃₋₈cycloalkyl wherein each of said C₁₋₆alkyl, C₃₋₈cycloalkyl, C₃₋₈heterocycloalkyl, C₁₋₆alkylaryl, C₁₋₆alkylheteroaryl or C₁₋₆alkyl-C₃₋₈cycloalkyl is optionally substituted with one or more substituents each independently selected from: OH, halogen, haloC₁₋₆alkyl, cyano and NH₂;
each of R₇ and R₈ are independently selected from:
- hydrogen;
- C₁₋₁₂alkyl optionally substituted with one or more substituents each independently selected from the group consisting of: OH, halogen, CN, NH₂, NH(R₉), N(R₉)₂, haloC₁₋₆alkyl, aryl, heteroaryl, 3-7 membered saturated ring and 5-7 membered partially saturated ring, each of said saturated or partially saturated ring optionally containing one or more heteroatoms selected from the group consisting of O, N and S and each of said aryl, heteroaryl, 3-7 membered saturated ring or 5-7 membered partially saturated ring being optionally substituted with one or more substituents each independently selected from: OH, halogen, C₁₋₆alkyl, haloC₁₋₆alkyl, CN, haloC₁₋₆alkoxy and C₁₋₆alkoxy;
- aryl or heteroaryl, each of said aryl or heteroaryl being optionally substituted with one or more substituents each independently selected from: OH, halogen, haloC₁₋₆alkyl, CN, haloC₁₋₆alkoxy and C₁₋₆alkoxy; and
- a 3-8 membered saturated or partially saturated cyclic or bicyclic ring optionally containing one or more heteroatoms each independently selected from the group consisting of: O, S and N, the 3-8 membered saturated or partially saturated cyclic or bicyclic ring being optionally substituted with one, two or more substituents each independently selected from the group consisting of: OH, halogen, CN, C₁₋₆alkyl, hydroxyC₁₋₆alkyl, C(O)OR₉, C(O)R₉, haloC₁₋₆alkyl, haloC₁₋₆alkoxy and C₁₋₆alkoxy;
or R₇ and R₈ form together with the nitrogen atom to which they are attached a cyclic amine selected from: aziridine, azetidine, pyrrolidine, piperidine, azepane, morpholine, thiomorpholine and piperazine each of said cyclic amine being optionally substituted with one or more substituents each independently selected from the group consisting of: OH, halogen, CN, C₁₋₆alkyl, hydroxyC₁-₆alkyl, haloC₁₋₆alkyl, haloC₁₋₆alkoxy and C₁₋₆alkoxy;
Ra, Rb, Rc and Rd are each independently selected from the group consisting of: hydrogen, halogen, CN, C₁₋₆alkyl, C₁-₆alkoxy, haloC₁₋₆alkyl, haloC₁-₆alkoxy, C(O)OR₉, C(O)R₉, NH₂, NH(R₉) and N(R₉)₂;
each R₉ is independently selected from C₁₋₆alkyl, haloC₁₋₆alkyl, C₁₋₆alkylaryl, C₁₋₆alkylheteroaryl and C₁₋₆alkyl-C₃₋₈cycloalkyl;
wherein said compound of general formula (I) is selected form the group consisting of:
- (3aR,10aR)-N-(3-chloro-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-N-(3,4-difluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-N-(3-(difluoromethyl)-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-N-(4-fluoro-3-(trifluoromethyl)phenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-6-chloro-N-(3-chloro-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-6-chloro-N-(3,4-difluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-6-chloro-N-(3-(difluoromethyl)-4-fluorophenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
- (3aR,10aR)-6-chloro-N-(4-fluoro-3-(trifluoromethyl)phenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide; and
- (3aR,10aR)-6-chloro-N-(4-fluoro-3-methylphenyl)-7-methyl-2-(2-oxo-2-(((R)-1,1,1-trifluoropropan-2-yl)amino)acetyl)-2,3,3a,4,10,10a-hexahydro-1H,7H-dipyrrolo[3,4-b:3',4'-f][1,4,5]oxathiazocine-8-carboxamide 5,5-dioxide;
or a pharmaceutically acceptable salt, tautomer, solvate or stereoisomer thereof.

2. A compound or a pharmaceutically acceptable salt, tautomer, solvate, or stereoisomer thereof as defined in claim 1 for medical use.

3. The compound or the pharmaceutically acceptable salt, tautomer, solvate, or stereoisomer thereof as defined in claim 2 for use in the treatment and/or prevention of an HBV infection and/or a condition related to an HBV infection, preferably said condition related to an HBV infection is selected from the group consisting of: chronic hepatitis B, HBV/HDV co-infection, HBV/HCV co-infection, HBV/HIV co-infection, inflammation, necrosis, cirrhosis, hepatocellular carcinoma, hepatic decompensation and hepatic injury from an HBV infection.

4. The compound or the pharmaceutically acceptable salt, tautomer, solvate, or stereoisomer thereof according to claim 2 or 3 for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection.

5. The compound or the pharmaceutically acceptable salt, tautomer, solvate, or stereoisomer thereof for use according to any one of claims 2-4, for use in combination with at least one further therapeutic agent, preferably said at least one further therapeutic agent is selected from the group consisting of: a therapeutic vaccine; an RNA interference therapeutic/antisense oligonucleotide; an immunomodulator; a STING agonist; a RIG-I modulator; a NKT modulator; an IL agonist; an interleukin or another immune acting protein; a therapeutic and prophylactic vaccine; an immune checkpoint modulator/inhibitor; an HBV entry inhibitor; a cccDNA modulator; an inhibitor of HBV protein espression; an agent targeting HBV RNA; a capsid assembly inhibitor/modulator; a core or X protein targeting agent; a nucleotide analogue; a nucleoside analogue; an interferon or a modified interferon; an HBV antiviral of distinct or unknown mechanism; a cyclophilin inhibitor; a sAg release inhibitor; a HBV polymerase inhibitor; a dinucleotide; a SMAC inhibitor; a HDV targeting agent; a viral maturation inhibitor; a reverse transcriptase inhibitor and an HBV RNA destabilizer or another small-molecule inhibitor of HBV protein expression; or a combination thereof; wherein said therapeutic vaccine is preferably selected from: HBsAG-HBIG, HB-Vac, ABX203, NASVAC, GS-4774, GX- 110 (HB-110E), CVI-HBV-002, RG7944 (INO-1800), TG-1050, FP-02 (Hepsyn-B), AIC649, VGX-6200, KW-2, TomegaVax-HBV, ISA-204, NU-500, INX-102-00557, HBV MVA and PepTcell; wherein said RNA interference therapeutic is preferably selected from: TKM-HBV (ARB-1467), ARB-1740, ARC-520, ARC-521, BB-HB-331, REP-2139, ALN-HBV, ALN-PDL, LUNAR-HBV, GS3228836 and GS3389404; wherein said immunomodulator is preferably a TLR agonist, preferably a TLR7, TLR8 or TLR9 agonist, preferably being selected from: RG7795 (RO-6864018), GS-9620, SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-pyrin-9-yl)propyl][3-(4-morpholinyl)propyl]amino}methyl)phenyl] acetate) and ARB-1598; wherein said RIG-I modulator is preferably SB-9200; wherein said IL agonist or other immune acting protein is preferably INO-9112 or recombinant IL12; wherein said immune checkpoint modulator/inhibitor is preferably BMS-936558 (Opdivo (nivolumab)) or pembrolizumab; wherein said HBV entry inhibitor is preferably Myrcludex B, IVIG-Tonrol or GC-1102; wherein said cccDNA modulator is preferably selected from: a direct cccDNA inhibitor, an inhibitor of cccDNA formation or maintenance, a cccDNA epigenetic modifier and an inhibitor of cccDNA transcription; wherein said capsid assembly inhibitor/modulator, core or X protein targeting agent, direct cccDNA inhibitor, inhibitor of cccDNA formation or maintenance, or cccDNA epigenetic modifier is preferably selected from: BAY 41-4109, NVR 3-778, GLS-4, NZ-4 (W28F), Y101, ARB-423, ARB-199, ARB-596, AB-506, JNJ-56136379, ASMB-101 (AB-V102), ASMB-103, CHR-101, CC-31326, AT-130 and RO7049389; wherein said interferon or modified interferon is preferably selected from: interferon alpha (IFN-α), pegylated interferon alpha (PEG-IFN-a), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta-la, peginterferon beta-la, interferon delta, interferon lambda (IFN-λ), peginterferon lambda-1, interferon omega, interferon tau, interferon gamma (IFN-γ), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA and interferon alpha biobetter; wherein said HBV antiviral of distinct or unknown mechanism is selected from: AT-61 ((E)-N-(1-chloro-3-oxo-1-phenyl-3-(piperidin-1-yl)prop-1-en-2-yl)benzamide), AT 130 ((E)-N-(1-bromo-1-(2-methoxyphenyl)-3-oxo-3-(piperidin-1-yl)prop-1-en-2-yl)-4-nitrobenzamide), analogues thereof, REP-9AC (REP-2055), REP-9AC' (REP-2139), REP-2165 and HBV-0259; wherein said cyclophilin inhibitor is preferably selected from: OCB-030 (NVP-018), SCY-635, SCY-575 and CPI-431-32; wherein said HBV polymerase inhibitor is preferably selected from: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir, preferably said tenofovir is in a salt form selected from: tenofovir disoproxil fumarate (Viread), tenofovir alafenamide fumarate (TAF), tenofovir disoproxil orotate (DA-2802), tenofovir disopropxil aspartate (CKD-390), AGX-1009, and CMX157; wherein said dinucleotide is preferably SB9200; wherein said SMAC inhibitor is preferably Birinapant; wherein said HDV targeting agent is preferably Lonafamib; wherein said HBV RNA destabilizer or other small-molecule inhibitor of HBV protein expression is preferably RG7834 or AB-452.

6. A pharmaceutical composition comprising the compound or the pharmaceutically acceptable salt, tautomer, solvate, or stereoisomer thereof as defined in claim 1, alone or in combination with at least one further therapeutic agent, and at least one pharmaceutically acceptable excipient, preferably said at least one further therapeutic agent is selected from the group consisting of: a therapeutic vaccine; an RNA interference therapeutic/antisense oligonucleotide; an immunomodulator; a STING agonist; a RIG-I modulator; a NKT modulator; an IL agonist; an interleukin or another immune acting protein; a therapeutic and prophylactic vaccine; an immune checkpoint modulator/inhibitor; an HBV entry inhibitor; a cccDNA modulator; an inhibitor of HBV protein espression; an agent targeting HBV RNA; a capsid assembly inhibitor/modulator; a core or X protein targeting agent; a nucleotide analogue; a nucleoside analogue; an interferon or a modified interferon; an HBV antiviral of distinct or unknown mechanism; a cyclophilin inhibitor; a sAg release inhibitor; a HBV polymerase inhibitor; a dinucleotide; a SMAC inhibitor; a HDV targeting agent; a viral maturation inhibitor; a reverse transcriptase inhibitor and an HBV RNA destabilizer or another small-molecule inhibitor of HBV protein expression; or a combination thereof; wherein said therapeutic vaccine is preferably selected from: HBsAG-HBIG, HB-Vac, ABX203, NASVAC, GS-4774, GX- 110 (HB-110E), CVI-HBV-002, RG7944 (INO-1800), TG-1050, FP-02 (Hepsyn-B), AIC649, VGX-6200, KW-2, TomegaVax-HBV, ISA-204, NU-500, INX-102-00557, HBV MVA and PepTcell; wherein said RNA interference therapeutic is preferably selected from: TKM-HBV (ARB-1467), ARB-1740, ARC-520, ARC-521, BB-HB-331, REP-2139, ALN-HBV, ALN-PDL, LUNAR-HBV, GS3228836 and GS3389404; wherein said immunomodulator is preferably a TLR agonist, preferably a TLR7, TLR8 or TLR9 agonist, preferably being selected from: RG7795 (RO-6864018), GS-9620, SM360320 (9-benzyl-8-hydroxy-2-(2-methoxy-ethoxy)adenine), AZD 8848 (methyl [3-({[3-(6-amino-2-butoxy-8-oxo-7,8-dihydro-9H-pyrin-9-yl)propyl][3-(4-morpholinyl)propyl]amino}methyl)phenyl]acetate) and ARB-1598; wherein said RIG-I modulator is preferably SB-9200; wherein said IL agonist or other immune acting protein is preferably INO-9112 or recombinant IL12; wherein said immune checkpoint modulator/inhibitor is preferably BMS-936558 (Opdivo (nivolumab)) or pembrolizumab; wherein said HBV entry inhibitor is preferably Myrcludex B, IVIG-Tonrol or GC-1102; wherein said cccDNA modulator is preferably selected from: a direct cccDNA inhibitor, an inhibitor of cccDNA formation or maintenance, a cccDNA epigenetic modifier and an inhibitor of cccDNA transcription; wherein said capsid assembly inhibitor/modulator, core or X protein targeting agent, direct cccDNA inhibitor, inhibitor of cccDNA formation or maintenance, or cccDNA epigenetic modifier is preferably selected from: BAY 41-4109, NVR 3-778, GLS-4, NZ-4 (W28F), Y101, ARB-423, ARB-199, ARB-596, AB-506, JNJ-56136379, ASMB-101 (AB-V102), ASMB-103, CHR-101, CC-31326, AT-130 and RO7049389; wherein said interferon or modified interferon is preferably selected from: interferon alpha (IFN-α), pegylated interferon alpha (PEG-IFN-α), interferon alpha-2a, recombinant interferon alpha-2a, peginterferon alpha-2a (Pegasys), interferon alpha-2b (Intron A), recombinant interferon alpha-2b, interferon alpha-2b XL, peginterferon alpha-2b, glycosylated interferon alpha-2b, interferon alpha-2c, recombinant interferon alpha-2c, interferon beta, interferon beta-la, peginterferon beta-la, interferon delta, interferon lambda (IFN-λ), peginterferon lambda-1, interferon omega, interferon tau, interferon gamma (IFN-γ), interferon alfacon-1, interferon alpha-nl, interferon alpha-n3, albinterferon alpha-2b, BLX-883, DA-3021, PI 101 (also known as AOP2014), PEG-infergen, Belerofon, INTEFEN-IFN, albumin/interferon alpha 2a fusion protein, rHSA-IFN alpha 2a, rHSA-IFN alpha 2b, PEG-IFN-SA and interferon alpha biobetter; wherein said HBV antiviral of distinct or unknown mechanism is selected from: AT-61 ((E)-N-(1 -chloro-3-oxo-1-phenyl-3-(piperidin-1-yl)prop-1-en-2-yl)benzamide), AT 130 ((E)-N-(1-bromo-1-(2-methoxyphenyl)-3 -oxo-3-(piperidin-1-yl)prop-1-en-2-yl)-4-nitrobenzamide), analogues thereof, REP-9AC (REP-2055), REP-9AC' (REP-2139), REP-2165 and HBV-0259; wherein said cyclophilin inhibitor is preferably selected from: OCB-030 (NVP-018), SCY-635, SCY-575 and CPI-431-32; wherein said HBV polymerase inhibitor is preferably selected from: entecavir (Baraclude, Entavir), lamivudine (3TC, Zeffix, Heptovir, Epivir, and Epivir-HBV), telbivudine (Tyzeka, Sebivo), clevudine, besifovir, adefovir (hepsera), tenofovir, preferably said tenofovir is in a salt form selected from: tenofovir disoproxil fumarate (Viread), tenofovir alafenamide fumarate (TAF), tenofovir disoproxil orotate (DA-2802), tenofovir disopropxil aspartate (CKD-390), AGX-1009, and CMX157; wherein said dinucleotide is preferably SB9200; wherein said SMAC inhibitor is preferably Birinapant; wherein said HDV targeting agent is preferably Lonafamib; wherein said HBV RNA destabilizer or other small-molecule inhibitor of HBV protein expression is preferably RG7834 or AB-452.

7. The pharmaceutical composition according to claim 6 for use in the treatment and/or prevention of an HBV infection and/or a condition related to an HBV infection, preferably said condition related to an HBV infection is selected from the group consisting of: chronic hepatitis B, HBV/HDV co-infection, HBV/HCV co-infection, HBV/HIV co-infection, inflammation, necrosis, cirrhosis, hepatocellular carcinoma, hepatic decompensation and hepatic injury from an HBV infection.

8. The pharmaceutical composition according to claim 6 or 7 for use in treating, eradicating, reducing, slowing or inhibiting an HBV infection in an individual in need thereof, and/or in reducing the viral load associated with an HBV infection in an individual in need thereof, and/or reducing reoccurrence of an HBV infection in an individual in need thereof, and/or inducing remission of hepatic injury from an HBV infection in an individual in need thereof, and/or prophylactically treating an HBV infection in an individual afflicted with a latent HBV infection.

9. A process for the synthesis of the compounds according to claim 1 comprising at least one of the following steps:
- reacting a compound of formula (1) wherein Ra is selected from the group consisting of Cl, F, CHF₂, CF₃ and CH₃, with a compound of formula (2) in the presence of an amine as 2,3,4,6,7,8,9,10-octahydropyrimido[1,2-a]azepine and in a solvent as ethanol to afford a compound of formula (3): 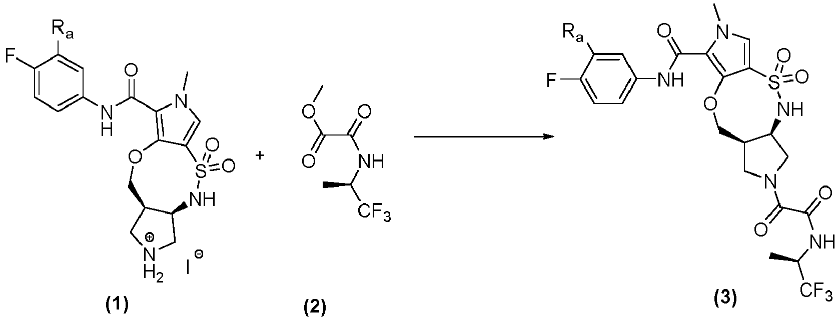
- reacting a compound of formula (3) wherein Ra is selected from the group consisting of Cl, F, CHF₂, CF₃ and CH₃ with sulfuryl dichloride in a solvent like dichloromethane to afford a compound of formula (4):
